# EUROPEAN PATENT SPECIFICATION

(11) **EP 2 385 979 B1**
(45) Date of publication and mention of the grant of the patent: **16.08.2017**
(21) Application number: 10702955.5
(22) Date of filing: 27.01.2010
(51) Int. Cl.: C12N 1/06, C12N 15/10, C12Q 1/68

(54) **SEQUENCE-SPECIFIC LARGE VOLUME SAMPLE PREPARATION METHOD AND ASSAY**
SEQUENZSPEZIFISCHES VERFAHREN ZUR VORBEREITUNG GROSSVOLUMIGER PROBEN UND ASSAY
PROCÉDÉ DE PRÉPARATION D'UN ÉCHANTILLON DE GRAND VOLUME SPÉCIFIQUE À UNE SÉQUENCE ET DOSAGE

(30) Priority: 28.01.2009 US 147862 P; 14.09.2009 US 242193 P
(43) Date of publication of application: 16.11.2011
(73) Proprietor: QIAGEN Gaithersburg, Inc., Gaithersburg, MD 20878 (US)
(72) Inventor: O'NEIL, Dominic, Alexandria, VA 22302 (US); UPTON, Karolina, Gaithersburg, Maryland 20878 (US); NAZARENKO, Irina, Gaithersburg, Maryland 20878 (US); DOSEEVA, Victoria, Rockville, MD 20855 (US); LOEFFERT, Dirk, D-40589 Düsseldorf (DE); FORBES, Thomas, Germantown, MD 20874 (US); WOLFF, John, Washington, DC 20009 (US); KOBAYASHI, Lori, Boonsboro, MD 21713 (US); RANGWALLA, Sameera, Gaithersburg, MD 20878 (US)
(74) Representative: von Renesse, Dorothea
(86) International application number: PCT/US2010/022264
(87) International publication number: WO 2010/088292

(56) References cited:
- WO-A1-93/10263
- US-A1- 2006 160 188
- US-B1- 6 291 166
- COHENFORD M A ET AL: "C-195. RAPID DETECTION OF CHLAMYDIA TRACHOMATIS FROM SPECIMENS COLLECTED FOR THE THINPREPR PAP TESTTM USING MOLECULAR BEACONS AND THE ROCHE LIGHTCYCLERTM" ABSTRACTS OF THE GENERAL MEETING OF THE AMERICAN SOCIETY FOR MICROBIOLOGY, THE SOCIETY, WASHINGTON, DC, US, vol. 101, 1 January 2001 (2001-01-01), page 195, XP001098006 ISSN: 1060-2011
- GENTECH DIAGNOSTICS: "Chlamydia DNA Test Kit" INTERNET CITATION, [Online] 6 June 2008 (2008-06-06), XP002578832 Retrieved from the Internet: URL:http://www.gentechin.com/chlamydiatest kit.htm> [retrieved on 2010-04-20]
- GENTECH DIAGNOSTICS: "Digene TM HBV Test Hybrid Capture TM II" INTERNET CITATION, [Online] 6 June 2008 (2008-06-06), pages 1-3, XP002560368 Retrieved from the Internet: URL:http://www.gentechin.com/hbvdnatestkit .htm> [retrieved on 2009-12-11]
- TAHA ET AL: "Universal Collection Medium (UCM)<(>R) is as suitable as the Standard Transport Medium (STM)<(>R) for Hybrid Capture II<(>R) (HC-2) assay" JOURNAL OF CLINICAL VIROLOGY, ELSEVIER, AMSTERDAM, NL LNKD- DOI:10.1016/J.JCV.2005.12.011, vol. 36, no. 1, 1 May 2006 (2006-05-01), pages 32-35, XP005367693 ISSN: 1386-6532
- DARWIN LING H ET AL: "Comparison of Digene hybrid capture 2 and conventional culture for detection of Chlamydia trachomatis and Neisseria gonorrhoeae in cervical specimens." JOURNAL OF CLINICAL MICROBIOLOGY FEB 2002 LNKD- PUBMED:11825985, vol. 40, no. 2, February 2002 (2002-02), pages 641-644, XP002578833 ISSN: 0095-1137
- NAZARENKO I ET AL: "A novel method of HPV genotyping using Hybrid Capture<(>R) sample preparation method combined with GP5+/6+ PCR and multiplex detection on Luminex<(>R) XMAP<(>R)" JOURNAL OF VIROLOGICAL METHODS, ELSEVIER BV, NL LNKD- DOI:10.1016/J.JVIROMET.2008.09.002, vol. 154, no. 1-2, 1 December 2008 (2008-12-01), pages 76-81, XP025680302 ISSN: 0166-0934 [retrieved on 2008-10-22]

## Description

### FIELD

The present disclosure relates to methods and assays for processing and preparing large volume biological samples in an efficient manner. The present disclosure also relates to methods and assays capable of selectively and rapidly isolating low concentrations of target nucleic acid molecules isolated from biological or clinical samples and suspended in a large volume of collection media.

### BACKGROUND

There is an inherent challenge to sample preparation from large volume clinical samples where target is present at a low concentration, such as cervical samples in liquid-based cytology media. Most solutions available in the market place involve method steps which include time consuming process steps that slow the processing of biological or clinical samples present in a large volume of media at low concentrations. For example, many solutions and preparation methods include centrifugation steps or nonspecific absorption of target samples on paramagnetic beads. Centrifugation steps, for example, may add one hour or more to sequence specific sample preparation protocols and methodology. In addition to being time consuming, both centrifugation and nonspecific absorption on paramagnetic beads require steps that oftentimes decrease assay throughput and generate a complex mixture of cellular components that may negatively influence subsequent applications. The present disclosure addresses these limitations by introducing a unique sample preparation protocol capable of identifying target nucleic acid molecules present at a low concentration and suspended in a large volume of media. By using the methods of the present disclosure, target nucleic acid molecules contained in an aqueous solution can be rapidly and selectively detected in a large volume setting.

There is also a need to provide novel and effective methods, compositions, and kits for determining target nucleic acid molecules in a rapid, cost-effective, and reliable manner in developing countries where access to medical care is not readily available. For instance, speed in obtaining results is particularly important in locations where individuals travel long distances to provide sample specimens for clinical analysis. In such locations, it is advantageous that results are obtained within several hours or the same day while the patient is still present to avoid loss to follow-up associated with traveling from home to the test site. The methods and assays of the instant disclosure meet these needs by allowing medical technicians, doctors, or other qualified individuals to secure samples from patients and rapidly and accurately identify disorders by target nucleic acid detection.

### SUMMARY

In an aspect, the disclosure relates to a large volume sample preparation method, the method comprising:
(a) suspending a biological sample in about 1 mL or more of a collection media;
(b) denaturing and lysing the biological sample by adding a denaturation agent and lysis buffer to the suspended biological sample;
(c) hybridizing a target nucleic acid molecule to at least one polynucleotide probe;
(d) capturing the hybridized target nucleic acid molecule on a support;
(e) washing the captured hybrid-support with wash buffer.

In an aspect, the disclosure relates to a large volume sample preparation method, the method comprising:
(a) obtaining a biological sample in about 1 mL or more of urine, blood, or serum;
(b) denaturing and lysing the biological sample by adding a denaturation agent and lysis buffer to the suspended biological sample;
(c) hybridizing a target nucleic acid molecule to at least one polynucleotide probe;
(d) capturing the hybridized target nucleic acid molecule on a support;
(e) washing the captured hybrid-support with wash buffer.

In an aspect, the disclosure relates to a large volume sample preparation method, the method comprising:
(a) suspending a biological sample in about 1 mL or more of a collection media;
(b) denaturing and lysing the biological sample by adding a denaturation agent and lysis buffer to the suspended biological sample;
(c) hybridizing a target nucleic acid molecule to at least one polynucleotide probe;
(d) capturing the hybridized target nucleic acid molecule on a support;
wherein the denaturing and lysing step (b) is complete in less than about 10 minutes and the combination of the hybridizing step (c) and the capturing step (d) is complete in less than about 25 minutes.

In an aspect, the disclosure relates to a large volume sample preparation method, the method comprising:
(a) suspending a biological sample in about 1 mL or more of a collection media or obtaining a biological sample in urine, blood, or serum;
(b) denaturing and lysing the biological sample by adding a denaturation agent and lysis buffer to the suspended biological sample;
(c) hybridizing a target nucleic acid molecule to at least one polynucleotide probe;
(d) capturing the hybridized target nucleic acid molecule on a support;
wherein the denaturing and lysing step (b) is complete in less than about 30 minutes and the combination of the hybridizing step (c) and the capturing step (d) is complete in less than about 30 minutes, and
10 copies or more of the target nucleic acid molecule are isolated in less than about 1 hour.

In an aspect, the disclosure relates to a large volume sample preparation assay, the assay comprising:
(a) suspending a biological sample in about 1 mL or more of a collection media;
(b) denaturing and lysing the biological sample by adding a denaturation agent and lysis buffer to the suspended biological sample;
(c) hybridizing a target nucleic acid molecule to at least one polynucleotide probe;
(d) capturing the hybridized target nucleic acid molecule on a support;
wherein the denaturing and lysing step (b) is complete in less than about 30 minutes and the combination of the hybridizing step (c) and the capturing step (d) is complete in less than about 30 minutes, 10 copies or more of the target nucleic acid molecule are isolated in less than about 1 hour, and the method does not include a centrifugation step.

In an aspect, the denaturing and lysing step (b) is complete in less than about 10 minutes and the combination of the hybridizing step (c) and the capturing step (d) is complete in less than about 25 minutes. In yet another aspect, the denaturing and lysing step (b) is complete in less than about 7.5 minutes and the combination of the hybridizing step (c) and the capturing step (d) is complete in less than about 22.5 minutes. In another aspect, the denaturing and lysing step (b) is complete in less than about 5 minutes and the combination of the hybridizing step (c) and the capturing step (d) is complete in less than about 15 minutes.

In an aspect, the disclosure relates to a sample preparation assay, the assay comprising:
(a) suspending a biological sample in about 0.25 mL to about 1.0 mL of a collection media or obtaining a biological sample in urine, blood, or serum;
(b) denaturing and/or lysing the biological sample by adding a denaturation agent and/or lysis buffer to the suspended biological sample;
(c) hybridizing a target nucleic acid molecule to at least one polynucleotide probe;
(d) capturing the hybridized target nucleic acid molecule on a support; and
(e) washing the captured hybrid-support with wash buffer.

In an aspect, the disclosure relates to a sample preparation assay, the assay comprising:
(a) obtaining a biological sample in about 0.25 mL to about 1.0 mL of a urine, blood, or serum;
(b) denaturing and/or lysing the biological sample by adding a denaturation agent and/or lysis buffer to the suspended biological sample;
(c) hybridizing a target nucleic acid molecule to at least one polynucleotide probe;
(d) capturing the hybridized target nucleic acid molecule on a support; and
(e) washing the captured hybrid-support with wash buffer.

In an aspect, the denaturing and/or lysing step (b) is complete in less than about 10 minutes and the combination of the hybridizing step (c) and the capturing step (d) is complete in less than about 25 minutes. In yet another aspect, the denaturing and/or lysing step (b) is complete in less than about 7.5 minutes and the combination of the hybridizing step (c) and the capturing step (d) is complete in less than about 22.5 minutes. In another aspect, the denaturing and /or lysing step (b) is complete in less than about 5 minutes and the combination of the hybridizing step (c) and the capturing step (d) is complete in less than about 15 minutes.

In an aspect, the disclosure relates to a method for detecting the presence of a low concentration of target nucleic acid molecule in a large sample volume, the method comprising:
(a) suspending the biological sample in about 0.25 mL to about 1.0 mL of a collection media;
(b) denaturing the target nucleic acid molecule in the biological sample;
(c) forming a double-stranded nucleic acid hybrid by contacting at least one polynucleotide probe with the target nucleic acid molecule; and
(d) forming a double-stranded nucleic acid hybrid-support complex by capturing the double-stranded nucleic acid hybrid on a support.

In another aspect, the disclosure relates to a method for detecting the presence of a low concentration of target nucleic acid molecule in a large volume, the method comprising:
(a) suspending the biological sample in about 0.25 mL to about 1.0 mL of a collection media;
(b) denaturing the target nucleic acid molecule in the biological sample;
(c) forming a double-stranded nucleic acid hybrid by contacting at least one polynucleotide probe with the target nucleic acid molecule;
(d) forming a double-stranded nucleic acid hybrid-support complex by capturing the double-stranded nucleic acid hybrid on a support; and
(e) washing the captured hybrid-support with wash buffer
wherein 10 copies or more of the target nucleic acid molecule are isolated in less than about 30 minutes.

In another aspect, the disclosure relates to a method for detecting the presence of a low concentration of target nucleic acid molecule in a large volume, the method comprising:
(a) suspending the biological sample in about 0.25 mL to about 1.0 mL of a collection media;
(b) denaturing the target nucleic acid molecule in the biological sample;
(c) forming a double-stranded nucleic acid hybrid by contacting at least one polynucleotide probe with the target nucleic acid molecule;
(d) forming a double-stranded nucleic acid hybrid-support complex by capturing the double-stranded nucleic acid hybrid on a support; and
(e) washing the captured hybrid-support with wash buffer
wherein method steps (a) - (e) do not include a centrifugation step.

A method for determining the presence of a target nucleic acid molecule in a sample, the method comprising:
(a) suspending a biological sample in about 0.25 mL to about 1.0 mL of a collection medium;
(b) denaturing the target nucleic acid molecule in the sample;
(c) forming a double-stranded nucleic acid hybrid by contacting at least one polynucleotide probe with the target nucleic acid molecule;
(d) forming a double-stranded nucleic acid hybrid-support complex by capturing the double-stranded nucleic acid hybrid on a support;
wherein the target nucleic acid molecule is identified in about 15 minutes to about 3 hours.

In another aspect, 10 copies or more of the target nucleic acid molecule are isolated in less than about 15 minutes, less than about 30 minutes, less than about 45 minutes, or less than about 1 hour.

In another aspect, 50 copies or fewer of a target nucleic acid molecule are detected over a time period of about 30 minutes to about 1 hour.

In an aspect, from about 10 to about 100 copies of the target nucleic acid molecule are capable of being identified in about 15 minutes to about 2 hours.

In an aspect, the large volume sample preparation method is performed on an automated, semi-automated, or fully automated platform.

In one aspect, the collection media comprises 0.5% to about 2.0% NP-40, about 0.10% to about 0.40% sodium deoxycholate, about 25 mM to about 75 mM Tris-HCl, about 10 mM to about 50 mM EDTA, about 50 mM to about 200 mM NaCl, and about 0.01% to about 0.10% sodium azide.

In another aspect, the collection media is selected from the group consisting of PRESERVCYT, STM, and SUREPATH.

In another aspect, the biological sample is obtained from urine, blood, or serum.

In an aspect, the denaturation step is complete in less than about 30 minutes.

In another aspect, the hybrid-capture step is complete in less than about 30 minutes.

In another aspect, all of the lysed cellular material remains in the sample preparation solution during the denaturation, hybridization, and capture methods steps. In another aspect, the target nucleic acid molecule is not separated from the reminder of the lysed biological material until wash step (e).

These and further aspects are explained in the following detailed description of the disclosure.

### BRIEF DESCRIPTION OF THE DRAWINGS

FIG. 1 shows a range of bead concentrations tested in 1 mL of clean PRESERVCYT collection media against 0, 10, 25, and 100 copies of *Neisseria gonorrhoeae* genomic DNA. 1X represents a bead concentration of 0.04% in 25 µl YT blocker.
FIG. 2 shows a hybrid/capture sample preparation step at 30 minutes and 60 minutes incubation using 1 mL of both clean and clinical PRESERVCYT and testing 0, 10, 100, and 1000 copies of *Neisseria gonorrhoeae* genomic DNA.
FIG. 3 shows a hybrid/capture sample preparation step with 30 minutes incubation at room temperature and 50°C using 1 mL of both clean and clinical PRESERVCYT and testing 0, 10, 100, and 1000 copies of *Neisseria gonorrhoeae* genomic DNA.
FIG. 4 shows a hybrid/capture sample preparation step at 30 minutes incubation at 50°C using either 1 mL clinical PRESERVCYT or 1 mL of urine (pH 6.5) as the collection media and testing 0, 10, 25, 100, 1,000, and 10,000 copies of *Neisseria gonorrhoeae* genomic DNA.
FIG. 5 shows large volume sample preparation using a lysis buffer containing Sarkosyl, DTT, and Tween 20 or Maas-Dalhoff lysis buffer.
FIG. 6 shows large volume sample preparation at 15 minutes and 30 minutes with detection of 25 and 100 copies of *Chlamydia trachomatis.*
FIG. 7 shows lysis in a large volume sample preparation protocol at 15 minutes and 30 minutes with detection of 25 and 100 copies of *Neisseria gonorrhoeae.*
FIG. 8 shows hybrid/capture step in a large volume sample preparation protocol at 15 minutes and 30 minutes with detection of 25 and 100 copies of *Neisseria gonorrhoeae.*
FIG. 9 shows an example of a 60 minute large volume sample preparation protocol.
FIG. 10 shows an example of a 30 minute large volume sample preparation protocol.

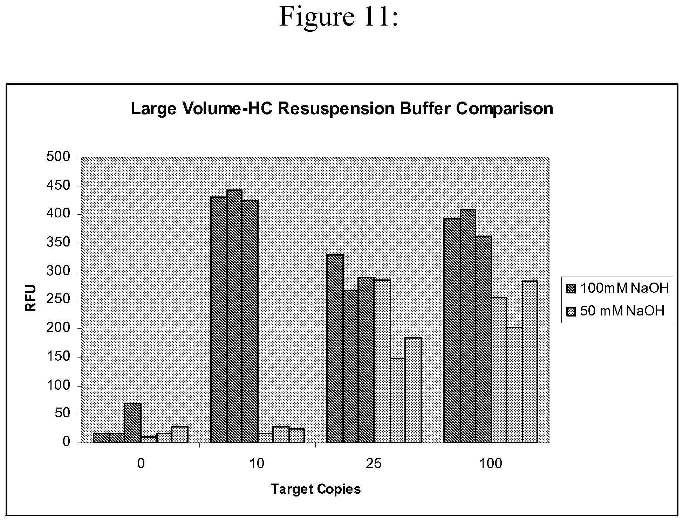

FIG. 11 shows large volume hybrid/capture with a resuspension buffer containing 50 mM NaOH or 100 NaOH tested with 0, 10, 25, and 100 copies of *Neisseria gonorrhoeae.*

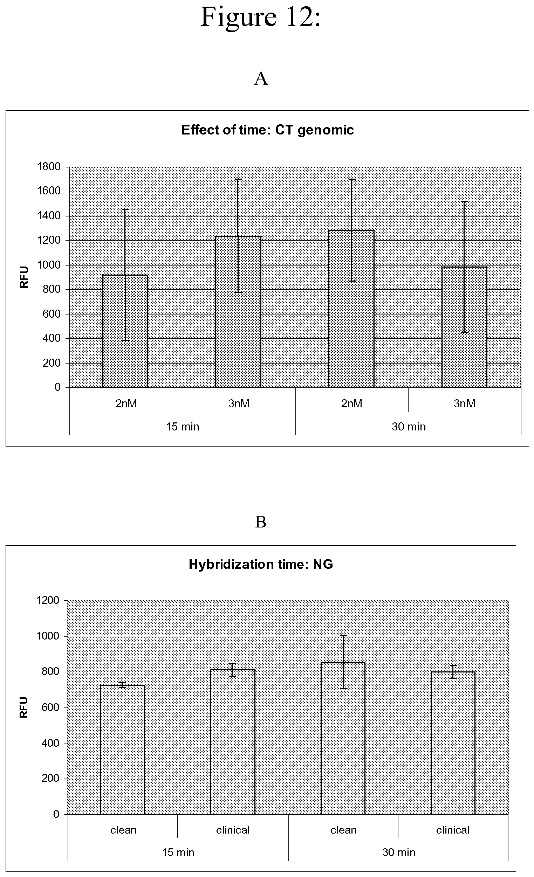

FIG. 12 shows (A) a large volume sample preparation involving a comparison between 2nM and 3nM synRNA concentraition at 15 minutes and 30 minutes incubation time by testing *Chlamydia trachomatis*; (B) a hybridization step comparison between clean and clinical PRESERVCYT at 15 minutes and 30 minutes by testing *Neisseria gonorrhoeae.*

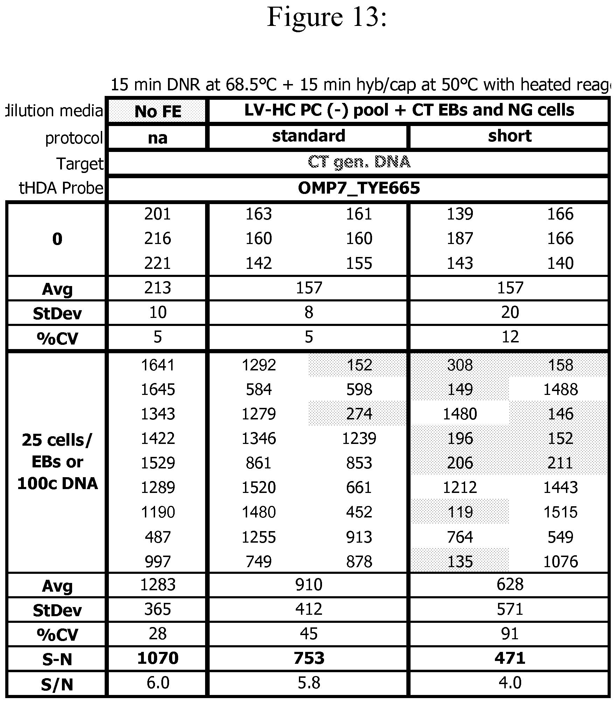

FIG. 13 shows a large volume sample preparation in PRESERVCYT media with a 15 minute denaturation step at 68.5°C and a 15 minute hybrid/capture step at 50°C with heated reagents. *Neisseria gonorrhoeae* and *Chlamydia trachomatis* cells were both tested.

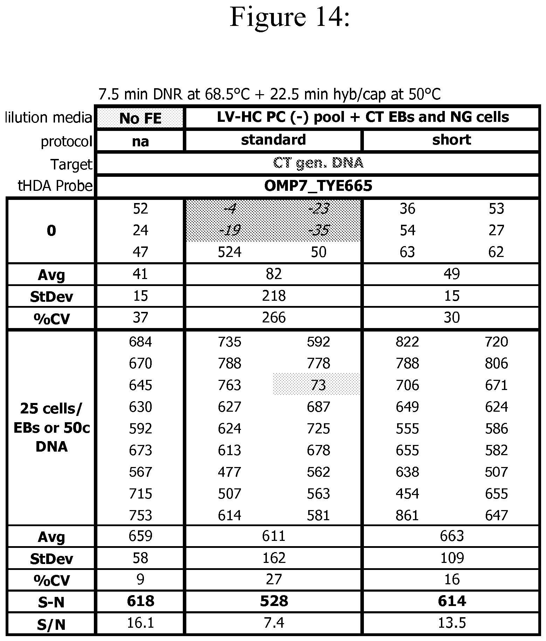

FIG. 14 shows a large volume sample preparation in PRESERVCYT media with a 7.5 minute denaturation step at 68.5°C and a 22.5 minute hybrid/capture step at 50°C with heated reagents. *Neisseria gonorrhoeae* and *Chlamydia trachomatis* cells were both tested.

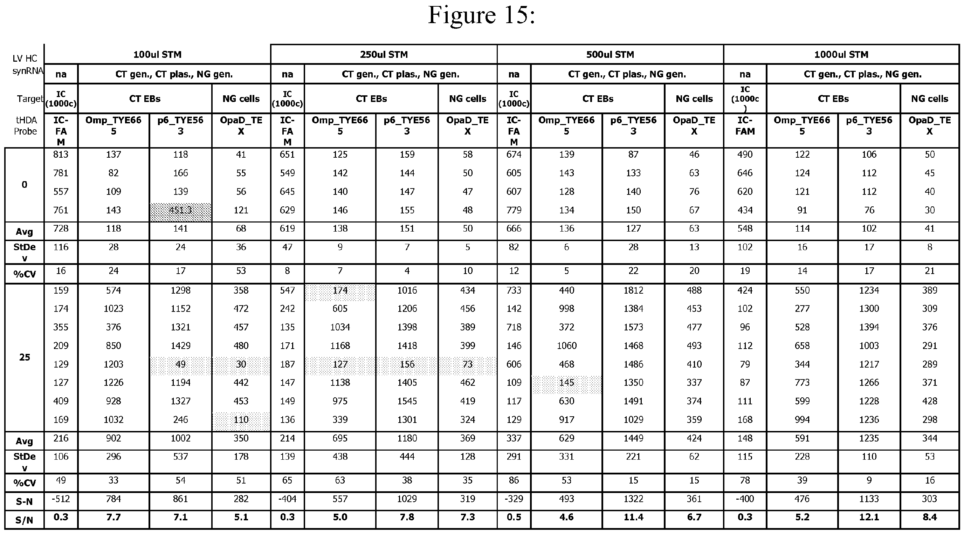

FIG. 15 shows a sample preparation in 100 µl, 250 µl, 500 µl, and 1000 µl STM media. *Neisseria gonorrhoeae* and *Chlamydia trachomatis* cells were both tested.

### DETAILED DESCRIPTION

The present disclosure relates to methods, compositions, reagents, and kits for rapidly and selectively determining the presence of a low concentration of target nucleic acid molecules in large volume or small volumes of collection medium. The methods, compositions, reagents, and kits may be used for clinical diagnostic purposes, including but not limited to the detection and identification of pathogenic organisms and the detection of a genetic predisposition to a particular disease.

### Sample Preparation

Large volume samples are those in which the target to be purified, enriched, or detected is in a large amount of sample, for example processing in about 0.5 ml, about 1 mL, and about 2 mL of sample or more. Generally, the target is diluted in the sample and as a result, difficult to purify, enrich, or detect. Using blood as an example, the detection of pathogens would be a large volume use of the sequence-specific method.

In another aspect, the sample preparation methods described herein are not limited to large volumes of sample. For example, a sample size of about 50 µl, about 100 µl, about 250 µl, about 100 µl to about 250 µl, or about 150 µl to about 250 µl can be used in conjunction with the sample preparation described herein. In another aspect, the smaller sample sizes of about 50 µl, about 100 µl, about 250 µl, about 100 µl to about 250 µl, or about 150 µl to about 250 µl may be analyzed on a microtiter plate in conjunction with the methods described herein.

In an aspect, a biological or clinical sample is collected or obtained, 1mL or more of a collection media is added to the sample, the suspended biological sample undergoes a lysis and/or denaturation step, after the lysis and/or denaturation steps are performed the biological sample undergoes a hybrid/capture step, and is subsequently washed. After the washing steps, the sample can be responded and the target nucleic acid molecule can be detected. In an aspect, the lysis and denaturation steps are completed within less than about 10 minutes and the hybrid/capture step is completed within less than about 25 minutes. In another aspect, the lysis and denaturation steps are completed within less than about 15 minutes and the hybrid/capture step is completed within less than about 15 minutes. In another aspect, a sample volume of 50 µl, about 100 µl, about 250 µl, about 100 µl to about 250 µl, or about 150 µl to about 250 µl may be used in the above method. In another aspect, such as the case with blood, serum, and urine, a biological or clinical sample is collected or obtained and there is no need to add collection media to the sample because the target nucleic acid molecule is contained within the urine, serum, or blood.

In an aspect, the large volume sample preparation method includes:
(a) adding a lysis buffer to a biological or cervical sample suspended in 1 mL or more of collection media;
(b) adding denaturation buffer to the biological or cervical sample suspended in 1 mL or more of collection media;
(c) hybridizing a target nucleic acid molecule to at least one polynucleotide probe;
(d) capturing the hybridized target nucleic acid molecule; and
(e) washing the captured hybrid-support with wash buffer.

In an aspect, after the wash step, the hybrid-capture support is resuspended in a resuspension buffer. In another aspect, after the large volume sample preparation protocol is complete, the target nucleic acid molecule is detected. In another aspect, after the large volume sample preparation protocol is complete, PCR is performed on the target nucleic acid molecules. The disclosed large sample volume preparation protocols may also be used with the methods for isolating and targeting nucleic acid molecules set forth in U.S. Provisional Application No: 12/605,540 and U.S. Patent Application No: 12/605,605. The disclosed sample volume preparation protocols may also be used in conjunction with the Hybrid Capture technology-based patents of U.S. Patent No. 4,732,847, U.S. Patent No. 4,865,980, and U.S. Patent No. 6,228,578. In another aspect, a sample volume of 50 µl, about 100 µl, about 250 µl, about 100 µl to about 250 µl, or about 150 µl to about 250 µl may be used in the above methods.

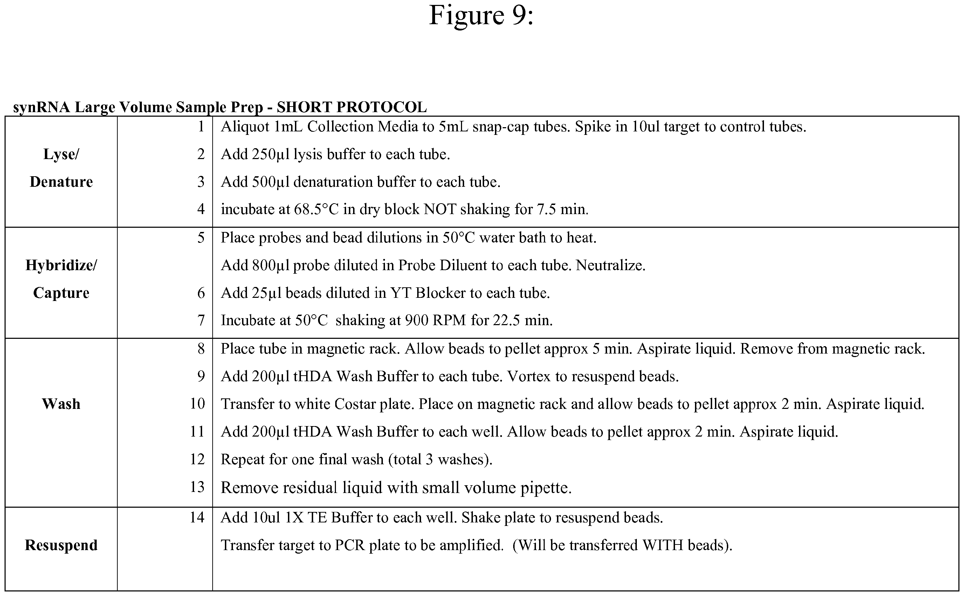

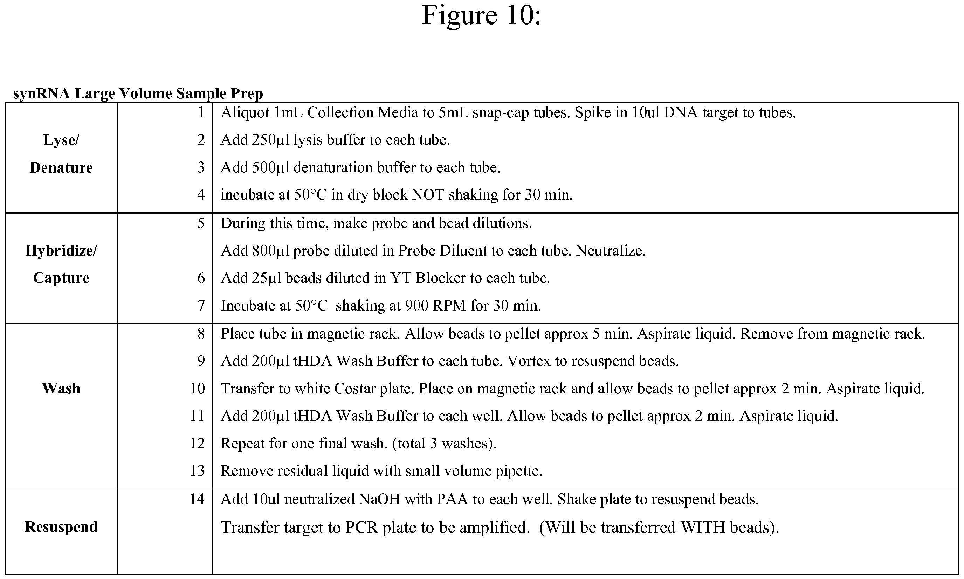

Without being limited, Figures 9 and 10 provide examples of large volume sample preparation protocols. In another aspect, the disclosed sample preparation methods in Figures 9 and 10 can have a sample volume of about 50 µl or more, about 100 µl or more, about 250 µl or more, about 100 µl to about 250 µl, or about 150 µl to about 250 µl, about 0.5 mL or more, about 1 mL or more, about 2 mL or more, about 3 mL or more, about 4 mL or more, about 5 mL or more, about 10 mL or more, or about 20 mL or more.

In an aspect, a clinical or biological sample may be processed using the disclosed large volume sample preparation methodology in conjunction with a semi-automated or fully automated assay or instrument. For example, a clinical or biological sample may be processed using the disclosed large volume sample preparation methodology in conjunction with the assays, methods, and instruments set forth in U.S. Patent Application No. 12/508,304, U.S. Patent Application No. 12/508,306, and U.S. Patent Application No. 12/622,131.

### Biological Sample

The sample preparation methods of the disclosure may be used to isolate or detect target nucleic acid molecule from samples, including, without limitation, a specimen or culture (*e.g.,* cellular, microbiological and viral cultures) including biological and environmental samples. Biological samples may be from an animal, including a human, fluid, solid (*e.g.,* stool) or tissue, as well as liquid and solid food and feed products and ingredients such as dairy items, vegetables, meat and meat by-products, and waste. Environmental samples include environmental material such as surface matter, soil, water and industrial samples, as well as samples obtained from food and dairy processing instruments, apparatus, equipment, utensils, disposable and non-disposable items.

In an aspect, the samples are biological samples including, but not limited to, cervical epithelial cells (*e.g.,* a sample obtained from a cervical swab), adenoid cells, anal epithelial cells, blood, saliva, cerebral spinal fluid, pleural fluid, milk, lymph, sputum and semen. The sample may comprise a double-stranded nucleic acid molecule or may comprise a single-stranded nucleic acid molecule. If a double-stranded nucleic acid molecule is present, it may be prepared for hybridization analysis by a variety of methods known in the art, *e.g.,* using alkali, using proteinase K/SDS, chaotropic salts. The process of preparing a double-stranded nucleic acid molecule for hybridization analysis generally involves converting it into a single-stranded nucleic acid molecule. This process is generally known as denaturation. However, it is also contemplated that a double-stranded nucleic acid molecule may be detected without denaturation, *e.g.,* through a triple-stranded construct.

The target nucleic acid molecule in a sample can be DNA or RNA or both DNA and RNA. The target nucleic acid molecule can be contained within a larger nucleic acid molecule. Detection of either the target nucleic acid molecule or the larger nucleic acid molecule containing the target nucleic acid molecule is contemplated by this disclosure.

The biological sample may comprise cervical cells, for example, human cervical cells. The sample can be collected with any method or device known in the art, including a chemically inert collection device such as a DACRON tipped swab. Other acceptable collection devices may be used including, but not limited to, cotton swab, cervical brush, flocked swab (a swab shaped like a DACRON swab but made with nylon fibers enabling collection of more cells and easier release of cells), cervical broom, mini broom, lavage, or any collection device often used in Pap smear testing.

In an aspect, the disclosed methods include collecting a sample from a woman over 30 years of age. The method can also include collecting a sample from a woman over 30 years via a Pap smear or comparable test. The sample collected by the Pap smear or comparable test can be a cervical cell sample.

Once the sample is collected, it may be placed in a sample tube. The tube can be sealed to prevent contamination. The collection device (swab, brush, etc.) may further contain a mechanism by which it can be moved once it is inside the sample tube. In one aspect, the collection device contains an insert that can be moved using a magnet. In one aspect, this insert comprises a metal. In another aspect, this insert comprises a paramagnetic material. In an aspect, the insert includes material ferromagnetic and diamagnetic materials. One advantage of moving the collection device once it is inside the sample tube is to avoid the collection device from making contact with any sample extraction or sample detection devices. Examples of a sample extraction device include pipettes, pipette tips, dropper bottles or other low tech extraction devices. Examples of sample detection devices include probes and probe tips.

In an aspect, the biological or clinical sample is not diluted. That is, the biological or clinical sample is collected from an individual and the disclosed large sample preparation methodology is immediately initiated. Evaluating the sample immediately after it is collected from an individual decreases the time necessary to identify a target nucleic acid molecule by the methods described herein and is beneficial in point of care venues, where same day results are given to the patient after the collection of a biological or clinical sample.

### Collection Medium

In an aspect, the large volume sample preparation method takes place in a collection medium. In another aspect, the biological sample is collected and stored in a collection medium. The collection medium has several functions including as a preservative medium to preserve nucleic acids and inhibit nucleases to prevent degradation of nucleic acids prior to analysis. In one aspect, the collection medium is detergent-based. Without being limited, examples of suitable collection media for use with the disclosure may be found in U.S. Patent Application No: 12/605,540 and U.S. Patent Application No: 12/605,605.

In one aspect, the detergent-based collection medium comprises, consists essentially of, or consists of 1.0% NP-40, 0.25% sodium deoxycholate, 50 mM Tris-HCl, 25 mM EDTA, 150 mM NaCl and 0.05% sodium azide. In another aspect the detergent-based collection medium comprises, consists essentially of, or consists of about 0.5% to about 2.0% NP-40, about 0.10% to about 0.40% sodium deoxycholate, about 25 mM to about 75 mM Tris-HCl, about 10 mM to about 50 mM EDTA, about 50 mM to about 200 mM NaCl, and about 0.01% to about 0.10% sodium azide. In other aspects the detergent-based collection medium comprises, consists essentially of, or consists of about 0.8% to about 1.5% NP-40, about 0.20% to about 0.40% sodium deoxycholate, about 30 mM to about 60 mM Tris-HCl, about 20 mM to about 40 mM EDTA, about 100 mM to about 200 mM NaCl, and about 0.025% to about 0.075% sodium azide. In yet another aspect the detergent-based collection medium comprises, consists essentially of, or consists of about 0.9% to about 1.2% NP-40, about 0.20% to about 0.30% sodium deoxycholate, about 30 mM to about 60 mM Tris-HCl, about 20 mM to about 30 mM EDTA, about 100 mM to about 150 mM NaCl, and about 0.04% to about 0.06% sodium azide.

In an aspect, the collection medium comprises, consists essentially of, or consists of NP-40 and EDTA. In another aspect, the collection medium comprises, consists essentially of, or consists of NP-40, EDTA, and sodium azide. In one aspect, the collection medium comprises, consists essentially of, or consists of sodium deoxycholate, EDTA, and sodium azide. In an aspect, the collection medium comprises, consists essentially of, or consists of about NP-40, sodium deoxycholate, EDTA, and sodium azide. In an aspect, the collection medium comprises, consists essentially of, or consists of NP-40, sodium deoxycholate, Tris-HCl, EDTA, and sodium azide.

In another aspect, the collection medium comprises, consists essentially of, or consists of 0.5% to about 2.0% NP-40 and 10 mM to about 50 mM EDTA. In another aspect, the collection medium comprises, consists essentially of, or consists of 0.5% to about 2.0% NP-40, 10 mM to about 50 mM EDTA, and about 0.01 % to about 0.10 % sodium azide. In one aspect, the collection medium comprises, consists essentially of, or consists of about 0.10% to about 0.40% sodium deoxycholate, 10 mM to about 50 mM EDTA, and about 0.01% to about 0.10% sodium azide. In an aspect, the collection medium comprises, consists essentially of, or consists of about 0.5% to about 2.0% NP-40, about 0.10% to about 0.40% sodium deoxycholate, 10 mM to about 50 mM EDTA, and about 0.01% to about 0.10% sodium azide. In an aspect, the collection medium comprises, consists essentially of, or consists of about 0.5% to about 2.0% NP-40, about 0.10% to about 0.40% sodium deoxycholate, about 25 mM to about 75 mM Tris-HCl, about 10 mM to about 50 mM EDTA, and about 0.01% to about 0.10% sodium azide. In certain embodiments, the medium comprises or consists essentially of 1% NP-40, 0.25% sodium deoxycholate, 50mM Tris-HCl, 25 mM EDTA, 150 mM NaCl and 0.09% sodium azide. This medium is often referred to herein and in the figures as Digene Collection Medium or DCM.

Samples may be collected in other known collection mediums and can be used in the methods described herein. Examples of other collection media include PRESERVCYT, SUREPATH, and STM (Sample/Specimen Transport Medium).

Certain collection media are nucleic acid specific. Samples collected in some of these media may require processing before the nucleic acids in the samples can be detected and analyzed. Various methods of processing samples (also known as preparing the samples) are known in the art. For example, cervical cell samples collected for cytological analysis in medium such as PRESERVCYT may be combined with a detergent-based lysis buffer followed by the addition of paramagnetic beads comprising nucleic acid binding surfaces. In addition, other cell samples collected in other known commonly available collection mediums may be combined with a detergent-based lysis buffer followed by the addition of paramagnetic beads comprising nucleic acid binding surfaces.

The detergent-based media may be mixed with PRESERVCYT, SUREPATH, or STM. In an aspect, a collection medium including 1% NP-40, 0.25% sodium deoxycholate, 50mM Tris-HCl, 25 mM EDTA, 150 mM NaCl and 0.09% sodium azide is mixed with PRESERVCYT, SUREPATH, or STM and added by a biological sample. In another aspect, a collection medium of about 75% PRESERVCYT, SUREPATH, or STM is mixed with about 25% of a collection medium including 1% NP-40, 0.25% sodium deoxycholate, 50mM Tris-HCl, 25 mM EDTA150 mM NaCl and 0.09% sodium azide. In another aspect, a collection medium of about 50% PRESERVCYT, SUREPATH, or STM is mixed with about 50% of a collection medium including 1% NP-40, 0.25% sodium deoxycholate, 50mM Tris-HCl, 25 mM EDTA, 150 mM NaCl and 0.09% sodium azide. In an aspect, PRESERVCYT, SUREPATH, or STM are diluted with water, which can improve the signal-to-noise ratio. Although detection in 100% SUREPATH, PRESERVCYT, or STM is feasible, both background and signal improves with dilution by a collection medium including 1% NP-40, 0.25% sodium deoxycholate, 50mM Tris-HCl, 25 mM EDTA, 150 mM NaCl and 0.09% sodium azide.

In an aspect, either "clean" or "clinical" collection media is used to suspend the biological sample. "Clean" collection media refers to collection media which does not contain a biological sample, such as a cell sample. In an aspect, target nucleic acid molecules may be suspended in "clean" collection media. In a "clean" collection media sample there is no clinical background present. "Clinical" collection media refers to collection media which contains a biological sample, such as a cell sample.

In an aspect, the biological or clinical sample is suspended in about 50 µl, about 100 µl, about 250 µl, about .5 mL, about .75 mL, about 1.0 mL, about 1.25 mL, about 1.5 mL, about 2.0 mL, about 2.5 mL, about 3.0 mL, about 5.0 mL, about 10 mL, about 15 mL, about 25 mL, about 30 mL, about 50 mL, or about 100 mL of an of the above collection media or mixtures thereof. In an aspect, the biological or clinical sample is suspended in about 50 µl or more, about 100 µl or more, about 250 µl, about .5 mL or more, about .75 mL or more, about 1.0 mL or more, about 1.25 mL or more, about 1.5 mL or more, about 2.0 mL or more, about 2.5 mL or more, about 3.0 mL or more, about 5.0 mL or more, about 10 mL or more, about 15 mL or more, about 25 mL or more, about 30 mL or more, about 50 mL or more, or about 100 mL or more of any the above collection media or mixtures thereof. In an aspect, the biological or clinical sample is suspended in about 50 µl, about 100 µl, about 250 µl, .5 mL, about .75 mL, about 1 mL, about 1.25 mL, about 1.5 mL, about 2.0 mL, about 2.5 mL, about 3.0 mL, about 5.0 mL, about 10 mL, about 15 mL, about 25 mL, about 30 mL, about 50 mL, or about 100 mL of PRESERVCYT, SUREPATH, STM, or a collection medium including about 0.5% to about 2.0% NP-40, about 0.10% to about 0.40% sodium deoxycholate, about 25 mM to about 75 mM Tris-HCl, about 10 mM to about 50 mM EDTA, about 50 mM to about 200 mM NaCl, and about 0.01% to about 0.10% sodium azide or mixtures thereof.

In another aspect, the biological sample to be analyzed and processed by the methods disclosed herein is present in a urine, serum, or blood sample in any of the above volumes. When the biological sample to be analyzed is present in urine, serum, blood, or any other bodily fluid, the sample may be collected and an aliquot taken for performing the large volume sample preparation analysis by any of the methods disclosed herein. In an aspect, urine has a pH of about pH 3.5, about pH 4.0, about pH 5, about pH 6; about pH 6.5, about pH 7.0, about pH 8.0, about pH 9.0, from about pH 4.5 to about pH 9.0, from about pH 6.0 to about pH 8.0, or from about pH 6.0 to about pH 7.0.

In an aspect, the sample preparation methods disclosed herein are applied to biological samples which have been previously prepared for diagnostic analysis. In one aspect, the biological sample to which the disclosed sample preparation methods are applied has been previously prepared for cytology analysis. In an aspect, the biological sample is collected from a patient and suspended in a media, such as SUREPATH, PRESERVCYT, STM, or a collection media including about 0.5% to about 2.0% NP-40, about 0.10% to about 0.40% sodium deoxycholate, about 25 mM to about 75 mM Tris-HCl, about 10 mM to about 50 mM EDTA, about 50 mM to about 200 mM NaCl, and about 0.01% to about 0.10% sodium azide. In another aspect, the biological sample to be analyzed and processed by the methods disclosed herein is present in a urine, serum, or blood sample. In an aspect, a portion of the suspended sample is evaluated for cytology purposes and an aliquot is removed for sample preparation purposes following the methodology disclosed herein. In another aspect, an aliquot of about 0.1 mL to about 0.5 ml, to about 0.5mL to about 1.0 mL, or about 1.0 mL to 2.0 mL is removed from the suspended biological sample and subject to the sample preparation methods described herein.

In an aspect, the biological sample is collected from a patient and suspended in about 1 mL or more, about 2 mL or more, about 5 mL or more, about 10 mL or more, or about 20 mL or more media. In another aspect, the biological sample is collected from a patient and suspended in about 1 mL of STM media, about 10 mL of SUREPATH media, or about 20 mL of PRESERVCYT media. In another aspect, after the biological sample is suspended in the above media, an aliquot is taken and subject to the sample preparation methods described herein. In an aspect, an aliquot of about 0.1 mL to about 0.5 ml, to about 0.5mL to about 1.0 mL, or about 1.0 mL to 2.0 mL is removed from the biological sample and subject to the sample preparation methods described herein

In an aspect, the sample is evaluated by the sample preparation methods described herein prior to cytology testing. In another aspect, the sample is evaluated by the sample preparation methods described herein after cytology testing.

In an aspect, the sample is prepared using a liquid based cytology (LBC) assay. LBC media can contain tissue fixatives such as alcohol and formalin which serve to stabilize the sample, inhibit bacterial growth, preserve cell morphology and diagnostic clusters, and assure the preparation of a tissue monolayer cytology slides. However, many compositions used to preserve biological samples, such as SUREPATH, contain alcohol or formalin which may be detrimental to analyzing nucleic acid molecules using conventional sample preparation methodology. In an aspect, the cytology slides contain cervical cell samples or any other biological sample capable of being evaluated. In an aspect, the SUREPATH media is used to prepare LBC sample. In addition to cytology preparation, LBC samples can be used for detection of disorders, such as common sexually transmitted pathogens, including Human Papillomavirus, *Neisseria gonorrhoeae,* and *Chlamydia trachomatis,* among others.

### Target Nucleic Acid Molecules

The target nucleic acid molecules include, without limitation, nucleic acid molecules found in specimens or cultures (*e.g*., cellular, microbiological and viral cultures) including biological and environmental samples. The target nucleic acid molecules may be found in biological samples from an animal, including a human, fluid, solid (*e.g.,* stool) or tissue, as well as liquid and solid food and feed products and ingredients such as dairy items, vegetables, meat and meat by-products, and waste. Target nucleic acid molecules may be found in environmental samples and include environmental material such as surface matter, soil, water and industrial samples, as well as samples obtained from food and dairy processing instruments, apparatus, equipment, utensils, disposable and non-disposable items.

The target nucleic acid molecules found in biological samples include, but not limited to cervical samples (*e.g.,* a sample obtained from a cervical swab) or cervical cell samples, adenoid cells, anal epithelial cells, blood, serum, saliva, cerebral spinal fluid, pleural fluid, milk, lymph, sputum, urine and semen. The target nucleic acid molecules may be from other viral, bacteria, mycobacteria or plasmodia, for example cytomegalovirus (CMV), herpes, HIV, H1N1, chlamydia, gonorrhea, *Neisseria gonorrhoeae* (GC), *Chlamydia trachomatis* (CT), *Trichomonas vaginalis, Staphylococcus aureus,* tuberculosis, SARS-associated coronavirus or influenza. In an aspect the target nucleic acid molecules are at least 70%, at least 80%, at least 85%, at least 90%, at least 95%, at least 96%, at least 97%, at least 98%, at least 98%, at least 99%, or 100% identical to nucleic acid molecules associated with any one of cervical samples (*e.g.,* a sample obtained from a cervical swab) or cervical cell samples, adenoid cells, anal epithelial cells, blood, saliva, cerebral spinal fluid, pleural fluid, milk, lymph, sputum, urine and semen, other viral, bacteria, mycobacteria or plasmodia, for example cytomegalovirus (CMV), herpes, HIV, H1N1, chlamydia, gonorrhea, *Neisseria gonorrhoeae, Chlamydia trachomatis*, *Trichomonas vaginalis*, *Staphylococcus aureus*, tuberculosis, SARS-associated coronavirus or influenza.

In one aspect, the target nucleic acid molecules are human papillomavirus (HPV) and include genetic variants of HPV. A variant includes polymorphisms, mutants, derivatives, modified, altered, or the like forms of the target nucleic acid. In one aspect, the target nucleic acid is an HPV nucleic acid. In another aspect, the HPV nucleic acid is HPV DNA of a high risk HPV type. In another aspect, the HPV nucleic acid is HPV RNA of a high risk HPV type. In another aspect the target nucleic acids are any one of high risk HPV types 16, 18, 26, 31, 33, 35, 39, 45, 51, 52, 56, 58, 59, 66, 68, and 82 or any one of low risk HPV types 6, 11, 40, 43, 53, 61, 67, 69, 70, 71, 72, 81, and 83.

In another aspect, a combination or set of nucleic acid molecules is targeted. For example, a set of target nucleic acid molecules can include high risk HPV types 16, 18, and 45. In an aspect, the set of nucleic acid molecules to be targeted include only high risk HPV types 16, 18, and 45. Further, a set of target nucleic acid molecules can comprise, consist essentially of, or consist of high risk HPV types 16, 18, and 45.

In another aspect, the target nucleic acid molecule is at least 70%, at least 80%, at least 85%, at least 90%, at least 95%, at least 96%, at least 97%, at least 98%, at least 98%, at least 99%, or 100% identical to nucleic acid molecules associated with any one of *Neisseria gonorrhoeae*, *Chlamydia trachomatis*, HPV, genetic variants of HPV, HPV DNA of a high risk HPV type, or HPV RNA of a high risk HPV type. In another aspect the target nucleic acids are at least 70%, at least 80%, at least 85%, at least 90%, at least 95%, at least 96%, at least 97%, at least 98%, at least 98%, at least 99%, or 100% identical to nucleic acid molecules associated with any one of high risk HPV types 16, 18, 26, 31, 33, 35, 39, 45, 51, 52, 56, 58, 59, 66, 68, and 82 or any one of low risk HPV types 6, 11, 40, 43, 53, 61, 67, 69, 70, 71, 72, 81, and 83.

Using methods of the present inventions, the target nucleic acid molecule may be present at concentrations less than about 1 pg per mL, less than about 0.75 pg per mL, less than 0.5 pg per mL, less than 0.25 pg per mL, and less than 0.2 pg per mL.

As noted previously, the target nucleic acid molecule may be DNA or RNA. When the target nucleic acid molecule is DNA, the probe is can be RNA and when the target nucleic acid is RNA, the probe is can be DNA. However, a DNA probe can be used with DNA target nucleic acid molecule and an RNA probe can be used with RNA target nucleic acid molecule.

### Denaturation and Lysis

After the sample is collected in a collection medium or obtained in, for example, blood, serum, or urine as described, the sample may be treated with a denaturation reagent to render the target nucleic acid molecule accessible to hybridization. In one aspect, the sample is denatured with an alkaline solution. Any alkali that can bring a solution pH to about pH 12, about pH 13, or about pH 14 may be used. Additionally, any alkali that can bring a solution pH to a range of about pH 12 to about pH 13, from about pH 12 to about pH 14, and from about pH 13 to about pH 14 can be used. Suitable concentrations of alkali include from about 1.0 N to about 2.0 N, from about 1.25 N to about 1.75 N, and from about 1.25 N to about 1.5 N, and about 1.5 N as well as any number within the recited ranges. Without being limited, suitable alkali include NaOH and KOH.

At room temperature, the sample treated with the denaturation reagent can be mixed by hand mixing or mechanical shaking at about 800 rpm, about 900 rpm, about 1000 rpm, between about 600 and about 1000 rpm, or between about 600 and 1200 rpm. In an aspect, the sample treated with the denaturation reagent is not shaken. The pH of the sample after addition of denaturation reagent can be about 14. In another aspect, the pH can be about pH 12 or pH 13. Such basic pH will both nick and denature a majority of the nucleic acid in the specimen. In addition, alkaline treatment can disrupt interactions between peptides and nucleic acids to improve accessibility of the target nucleic acid and degrade protein.

Alkaline treatment of protein effectively homogenizes the specimen to ensure reproducibility of analysis results for a given sample. It can also reduce the viscosity of the sample to increase kinetics, homogenize the sample, and reduce background by destroying any endogenous single stranded RNA nucleic acids, DNA-RNA hybrids or RNA-RNA hybrids in the sample. It also helps inactivate enzymes such as RNases and DNases that may be present in the sample. One skilled in that art would appreciate that if RNA is the target nucleic acid (as opposed to DNA), different reagents may be preferable including, but not limited to phenol extraction and TCA/acetone precipitation, and guanidinium thiocyanate-phenol-chloroform extraction.

Other methods of denaturation may be employed such as utilizing a heating step, for example, heating the sample to about 95°C to separate the strands of nucleic acid. Enzymes such as helicase may be used as well.

In one aspect, denaturation buffer, such as NaOH, is added to the sample and heated. In another aspect, 1.5 N to 2.0 N NaOH is added to the sample and heated. The sample with denaturation reagent may be heated to about 60°C to about 80°C for about less than 30 minutes, to about 65°C to about 75°C for about less than 30 minutes, to about 67°C to about 70°C for about less than 30 minutes, 68.5°C for about less than 30 minutes; or to about 70°C for about less than 30 minutes, or any number within the recited ranges. In another aspect, the sample with denaturation reagent is heated to about 60°C to about 80°C for about 10 to about 30 minutes, or to about 65°C to about 75°C for about 10 to about 30 minutes, to about 67°C to about 70°C for about 10 to about 30 minutes, to about 68.5°C for about 10 to about 30 minutes, or to about 70°C for about 10 to about 30 minutes, or any number within the recited ranges. In an aspect, the sample may be heated in denaturation reagent in the above conditions for about 5 to about 30 minutes, about 10 to about 40 minutes, about 20 minutes to about 40 minutes, or about 5 minutes, about 7.5 minutes, about 10 minutes, about 15 minutes, about 20 minutes, or about 30 minutes, or any number within the recited ranges. In yet another aspect, the above incubation and temperature times may be completed with or without shaking.

In an aspect, the denaturation step is performed at about 68.5°C for about 5 - 30 minutes; at about 68.5°C for about 5 - 15 minutes; at about 68.5°C for about 5 - 10 minutes; and about 68.5°C for about 7.5 minutes with or without shaking. In another aspect, the denaturation step is performed at two temperatures: 67.5°C for about 7.5 min and 60.0°C for about 12.5 minutes.

In an aspect, any lysis buffer capable of lysing cells or biological material may be used. In another aspect, the lysis buffer contains Sarkosyl, DTT, and Tween. In another aspect, the lysis buffer comprises, consists of, or consists essentially of about 7.5% sarkosyl, about 2.5% NP-40 and about 10 mM DTT. In another aspect, the lysis buffer comprises, consists of, or consists essentially of about 5.0% to about 10% sarkosyl, about 1.0 to about 5.0% NP-40, and about 1 mM to about 20 mM DTT. In another aspect, the lysis buffer comprises, consists of, or consists essentially of about 6.0% to about 8% sarkosyl, about 2.0 to about 3.0% NP-40, and about 5 mM to about 15 mM DTT. Maas-Dalhoff lysis buffer can also be used.

In an aspect, the biological or clinical sample can undergo a lysis step without removal of the extracted cellular material. In an aspect, the extracted or lysed cellular material is present during the lysis and/or denaturation step and hybrization/capture step and is first removed with washing. Additionally, in some aspects, the disclosed methods and assays are performed with unpurified biological or clinical sample. Accordingly, the disclosed methods and assays performed with unpurified biological or clinical samples can contain, for example, creams, lotions, and antifungals, cellular material and other impurities. Performing the disclosed methods on previously unpurified biological or clinical samples decreases the time necessary to detect target nucleic acid molecules under situations where the target is present in low concentrations. Decreasing the time necessary to detect target nucleic acid molecules is particularly useful when it is desirable to reach rapid identification of a disorder or disease, such as in developing countries where access to medicine and medical equipment may be sparse.

### Hybridization and Binding of Probes

In an aspect, after the sample containing the nucleic acid undergoes a lysis or denaturation step, the sample can be contacted with one or more polynucleotide probes under a condition sufficient for the one or more polynucleotide probes to hybridize to the target nucleic acid in the sample to form a double-stranded nucleic acid hybrid. The probe can be full length, truncated, or synthetic DNA or full length, truncated, or synthetic RNA ("syn RNA"). If the target nucleic acid is DNA, then the probe may be RNA and if the target nucleic acid is RNA, then the probe may be DNA. Preferably, the one or more polynucleotide probes are diluted in a probe diluent that also can act as a neutralizing hybridization buffer (to neutralize the basic denaturation reagent).

The probe diluent used for DNA or RNA probes will differ due to the different requirements necessary for DNA versus RNA stability. For example, if the probes are RNA, it is preferable to neutralize the sample first and than add the probe or alternatively, add the RNA probe and neutralizing agent (probe diluent) to the sample at the same time as NaOH can destroy RNA. The probe diluent can be used to dissolve and dilute the probe and also help restore the sample to about a neutral pH, *e.g*., about pH 6 to about pH 9, to provide a more favorable environment for hybridization. Sufficient volume of probe diluent, preferably one-half volume of the sample, may be used to neutralize the base-treated sample.

In an aspect, the probe diluent comprises a buffer, polyacrylic acid, NaOH and sodium azide. The probe diluent may comprise acetic acid. In one aspect, the probe diluent comprises 2.2 M BES (N,N-Bis(2-hydroxyethyl)-2-aminoethanesulfonic acid), 2.6% polyacrylic acid (PAA), 0.7 N NaOH and 0.05% sodium azide. The probe diluent may contain from about 1.2 M to about 2.6 M BES, from about 1.5 M to about 2.5 M BES; from about 1.75 M to about 2.25 M BES; from about 2 M to 2.4 M BES, or about 2.2 M BES, as well as any number within the recited ranges. In one aspect the probe diluent may contain from about 2% to about 3.0% PAA or, as well as any number within the recited ranges. In another aspect, the PAA concentration is from about 2.2% to about 2.7%. In yet another aspect, the PAA concentration is about 2.6%. In a further aspect the probe diluent may contain from about 0.6 N to about 0.8 N NaOH, for example, about 0.7 N NaOH. The concentration of NaOH generally increases as the amount of BES increases.

For full length probes, a heated alkaline solution may be added to the sample, then probe diluent may be added to the sample at room temperature, and then the sample may be reheated. Such a process can inhibit secondary structure from forming. Antibodies tend to irreversibly bind to structures with secondary structure. When using non-full length probes such as truncated or synthetic probes, heating the solutions or sample may not be necessary because secondary structures issues are not present. In an aspect, the sample is not heated when used with truncated or synthetic probes.

In an aspect, after treatment with the denaturation reagent, an aliquot of neutralization buffer, in an aspect the probe diluent described, in which the one or more probes are dissolved, can be added to the sample under appropriate conditions to allow hybridization or binding of the probe and the target nucleic acid to occur. The neutralization buffer may contain a single buffering salt. In an aspect, the neutralization buffer does not contain more than a single buffering salt. The hybridization condition is sufficient to allow the one or more polynucleotide probes to anneal to a corresponding complementary nucleic acid sequence, if present, in the sample to form a double-stranded nucleic acid hybrid.

Hybridization conditions suitable for the particular probes and diluents described herein are employed. The probes and sample nucleic acids can be incubated for a hybridization time, for example, at least about 5 to about 15 minutes, about 10 to about 20 minutes, about 10 to about 30 minutes, about 20 to about 30 minutes, about 20 to about 45 minutes, about 30 to about 1 hour, about 1 hour to about 2 hours, about 2 hours to about 4 hours, about 4 hours to about 24 at hybridization temperature of about 20°C, about 25°C, about 35°C, about 40°C, about 45°C, about 50°C, about 55°C, about 60°C, and about 65°C as well as any number within the recited ranges sufficient to allow the one or more polynucleotide probes to anneal to a corresponding complementary nucleic acid sequence. The samples may be incubated with or without shaking at the above temperatures and times.

Hybridization conditions suitable for the particular probes and diluents described herein are employed. The probes and sample nucleic acids can be incubated for a hybridization time, for example, at least about 5 to about 15 minutes, about 10 to about 20 minutes, about 10 to about 30 minutes, about 20 to about 30 minutes, about 20 to about 45 minutes, about 30 to about 1 hour, about 1 hour to about 2 hours, about 2 hours to about 4 hours, about 4 hours to about 24 at a hybridization temperature of about 20°C to about 25°C, about 35°C to about 40°C, about 45°C to about 50°C, about 55°C to about 60°C, and about 65°C to about 70°C as well as any number within the recited ranges sufficient to allow the one or more polynucleotide probes to anneal to a corresponding complementary nucleic acid sequence. The samples may be incubated with or without shaking at the above temperatures and times.

Without being limited, stringent hybridization conditions may be controlled by increasing the temperature, increasing the ionic conditions to above 0.5M (for example, NaCl), or reducing the concentration of PAA. As a non-limiting example, stringent hybridization conditions may include performing a hybridization reaction at elevated temperatures, such as of at least about 65°C, at least about 68.5°C, between about 67°C to about 70°C , and between about 69°C to about 70°C. Stringent hybridization conditions may also include elevated temperatures, such as of at least about 65°C, at least about 68.5°C, and between about 67°C to about 70°C.

In an aspect, the hybridization and/or capture step is completed at about 50°C in about 15 to about 25 minutes; at about 50°C in about 20 to about 25 minutes; or at about 50°C in about 22.5 minutes. In an aspect, the hybridization/capture is incubated with or without shaking.

In a non-limiting aspect, the probe is capable of hybridizing or binding to nucleic acid molecules at least 70%, at least 80%, at least 85%, at least 90%, at least 95%, at least 96%, at least 97%, at least 98%, at least 98%, at least 99%, or 100% identical to nucleic acid molecules associated with *Neisseria gonorrhoeae*, *Chlamydia trachomatis*, HPV, genetic variants of HPV, HPV DNA of a high risk HPV type, or HPV RNA of a high risk HPV type, or any one of high risk HPV types 16, 18, 31, 33, 35, 39, 45, 51, 52, 56, 58, 59, 66, 68, and 82 or any one of low risk HPV types 6, 11, 40, 43, 53, 61, 67, 69, 70, 71, 72, 81, and 83. In another aspect, the probe is complementary to HPV, genetic variants of HPV, HPV DNA of a high risk HPV type, HPV RNA of a high risk HPV type, or any one of high risk HPV types 16, 18, 31, 33, 35, 39, 45, 51, 52, 56, 58, 59, 66, 68, and 82 or any one of low risk HPV types 6, 11, 40, 43, 53, 61, 67, 69, 70, 71, 72, 81, and 83.

In an aspect, an oil or oil-type substance, such as silicone oil, is added to the sample prior to heating. In one aspect, an oil or oil-type substance is added to the sample prior to heating and the sample is examined on an automated platform, such as, for example, those described in U.S. Application No. 12/605,605, U.S. Patent Application No. 12/508,304, U.S. Patent Application No. 12/508,306, and U.S. Patent Application No. 12/622,131. The oil may have a viscosity of about 0.5 Cst to about 20 Cst, about 1.0 Cst to about 10 Cst, or about 2.0 Cst to about 5 Cst. In an aspect, the volume is about 5 Cst. In an aspect about 10 µl to about 45 µl of the above silicone oil is added to 1 mL or more of collection media and evaluated on an automated platform. One advantage of adding oil is that the sample is heated more evenly.

In one aspect, the sample is suspended in collection medium, the target nucleic acid is denatured with a denaturation reagent, and hybridized to nucleic acid probes suspended in a neutralizing buffer. In another aspect the neutralizing buffer is the probe diluent of the present invention. The probe diluent can comprises 2.2 M BES (*N*,*N*-bis(2-hydroxyethyl)-2-aminoethanesulfonic acid), 2.6% polyacrylic acid, 0.7 N NaOH and 0.05% sodium azide.

### Capture

After the probes hybridize to the target nucleic acid molecule and form a double-stranded nucleic acid hybrid, the hybrid may be captured by a molecule that is specific for the double-stranded nucleic acid hybrid. Molecules specific for the double stranded nucleic acid hybrids include, but are not limited to, monoclonal antibodies, polyclonal antibodies, proteins such as but not limited to RNAse H, nucleic acids including but not limited to aptamers, or sequence specific nucleic acids. Aptamers are short stretches of random sequences that are successively selected from a library of sequences by hybridizing to a target, amplifying the hybridized aptamers, and repeating the selection process. In one aspect the molecule specific for the double stranded nucleic acid hybrid is captured by an antibody, known as an anti-hybrid antibody.

In one aspect, an anti-hybrid antibody is immobilized onto a support using techniques that are standard in the art. Examples of suitable supports include covalent linkages or adsorption, for example, protein-protein interactions, protein-G beads, biotin-streptavidin interaction, EDAC to link to a carboxyl or tosyl group, etc., or hybridization directly onto the solid support using, for example, sequence specific nucleic acids in an affinity column.

Supports include but are not limited to beads, paramagnetic, diamagnetic, ferromagnetic, ferromagnetic, and diamagnetic beads, columns, plates, filter paper, polydimethylsiloxane (PDMS), and dipsticks. Any support can be used as long as it allows extraction of the liquid phase and provides the ability to separate out bound and unbound antibodies. Paramagnetic beads are particularly useful in that they can be left in the solution and the liquid phase can be extracted or decanted, if a magnetic field is applied to immobilize the beads. Beads that are small and have a high surface area may be used, such as beads about 1 µm in diameter. Other beads that employ charge switching or silica capture (as opposed to magnetic fields) may be used as well.

The hybrids can be incubated with the anti-hybrid antibody attached to the support for a sufficient amount of time to allow capture of the double-stranded nucleic acid hybrids by the immobilized anti-hybrid antibodies. In an aspect, the support is a bead.

The anti-hybrid antibody may be monoclonal or polyclonal. In one aspect the antibody is monoclonal. In another aspect, the antibody is coupled to support by an 1-ethyl-3-[3-dimethylaminopropyl] carbodiimide hydrochloride (EDAC) linker. In one aspect, the support is a polystyrene bead. In an aspect, the support or bead coupled to the antibody is diluted in a bead dilution buffer. The bead dilution buffer is helpful in minimizing protein denaturation on the bead. One example of a bead dilution buffer including 6% casein, 100 mM Tris-HCl, 300 mM NaCl, and 0.05% sodium azide.

In an aspect, the beads coated with the anti-hybrid antibody are incubated with the sample at about 45°C to about 55°C for about 30 minutes and about 50°C to about 60°C for about 30 minutes. In an aspect, the incubation time can range from about 5 minutes to about 60 minutes, from about 15 minutes to about 45 minutes, from about 20 minutes to about 40 minutes, or any number within the recited ranges. In an aspect, the incubation time is about 10 minutes, about 15 minutes, about 20 minutes, about 22.5 minutes, about 25 minutes, about 30 minutes, or about 45 minutes at between 45 °C and 55°C with or without shaking. In another aspect, the incubation takes place at about 22.5 minutes at 50°C without shaking.

Following capture of the target nucleic acid/probe hybrid as described above, the captured hybrid may be separated from the rest of the sample by washing away of non-captured nucleic acids.

### Conjugation

In an aspect, another step in the large volume sample preparation method can involve providing a second antibody that is also specific for double stranded nucleic acids hybrids or alternatively is specific for the first antibody. The second antibody, if present, may be detectably labeled, either directly or indirectly, and may be a monoclonal or polyclonal antibody. In an aspect, the second antibody is monoclonal. In another aspect, the second antibody is directly labeled with a detectable marker and is monoclonal. The second antibody is used to detect the presence of double-stranded nucleic acid hybrids. In one aspect, the second antibody has a label that must react with a substrate to provide a signal that can be detected. The second antibody may be dissolved in a suitable buffer. In one aspect the buffer comprises 100 mM TrisHCl, pH 7.4, 0.5 M NaCl, 0.1 mM ZnCl₂, 1.0 mM MgCl₂, 0.25% Tween 20, 0.2 mg/mL RNase A, 4% hydroxypropyl-b-cyclodextrin (cyclodextrin), 30% bead dilution buffer as discussed previously, 0.05% goat IgG, 0.05% sodium azide.

In an aspect, the conjugation reaction takes place at room temperature. In an aspect, the conjugation reaction takes place at room temperature for between about 1 hour and about 2 hours. In another aspect, the conjugation reaction takes place at room temperature for about 2 hours. In another aspect the conjugation reaction takes place at about 37°C, about 45°C, or about 50°C. In an aspect the conjugation reaction takes place at about 37°C, about 45°C, or about 50°C, from about 35°C to about 40°C, or from about 40°C to about 50°C for between about 15 minutes and about 30 minutes. In an aspect the conjugation reaction takes place at about 37°C, about 45°C, or about 50°C for between about 20 minutes and 40 minutes. In another aspect the conjugation reaction takes place at about 45°C for about 30 minutes.

It will be understood by those skilled in the art that any detectable label such as, but not limited to, an enzyme, radioactive molecule, fluorescent molecule, or metal particle such as gold particle can be used. In certain aspects, the detectable label is alkaline phosphatase. Methods of conjugating a label to an antibody are known. For example, an antibody can be reduced with dithiothreitol (DTT) to yield monovalent antibody fragments. The reduced antibody can then be directly conjugated to maleinated alkaline phosphatase by the methods of Ishikawa et al., J. Immunoassay 4:209-237 (1983) and Means et al., Chem. 1: 2-12 (1990), and the resulting conjugate can be purified by HPLC. The conjugate may also be purified using any type of size-exclusion chromatography. One benefit of purification is that the conjugates of one protein to one antibody can be separated from those conjugates with other ratios of protein to antibody.

In another aspect, the double-stranded nucleic acid hybrids can be detected with a second anti-hybrid antibody that is not directly labeled. For example, the second antibody can be a mouse immunoglobulin that is detected by a labeled goat anti-mouse antibody.

### Wash

In an aspect, following hybridization and capture, the sample may be washed with a wash buffer. The wash buffer may contain one or more detergents or may be free of a detergent. If the wash buffer contains a detergent, the detergent may be an ionic or a non-ionic detergent. One example of a non-ionic detergent is Triton-X. The detergent may be present in the wash buffer at a concentration of about 0.05% to about 1.5%, or from about 0.075% to about 1.0%, or from about 0.1% to about 0.75%, or about 0.5% or any number within the recited ranges. One example of a suitable wash buffer comprises 40 mM Tris, pH 8.2, 100 mM NaCl, 0.5% Triton-X 100 and 0.05% sodium azide. In another aspect, the wash buffer is from about .5 - 2 mM Tris, from about 0.02 - 0.10% sodium azide, with a pH from about 7.6 - about 8.4. In another aspect, the wash buffer is about 1 mM Tris, about 0.09% sodium azide, with a pH from about 7.6 - about 8.4.

The sample may be washed with the wash buffer from one to ten times, or from three to seven times, or from four to six times, or two, three, four, five times, or any number within the recited ranges. The sample may also be washed with a single wash buffer or with multiple wash buffers. Each wash may use the same wash buffer or a different wash buffer. For example, a detergent-containing wash buffer may be used for one wash while a detergent-free wash buffer may be used for another wash. In an aspect, one of the wash buffers does not include Triton.

One benefit of the detergent-containing wash buffer is the positive effects on bead behavior when compared to detergent-free wash buffers. The detergent-containing wash buffer allows for rapid, efficient, and resilient binding of the beads to the magnetic field. Binding of the beads to the magnetic field is strong enough that beads remain bound through physical inversion and decanting. While detergent-free wash buffers generally do not allow for physical inversion without bead loss, they may be used for other purposes. One example of the use of a detergent-free wash buffer is to remove or dilute a detergent in the sample thereby reducing any likely detection problems.

### Detection

In an aspect, the captured target nucleic acid molecule may be identified by a detection device or detection method. Any detection device capable of detecting target nucleic acid molecules may be used in conjunction with the sample preparation methods disclosed herein. Methods for detecting various labels are known in the art. For example, colorimetry, radioactive, surface plasmon resonance, or chemiluminescence methods are described by *e.g.,* Coutlee et al., J. Clin. Microbiol. 27:1002-1007 (1989). In an aspect, the captured target nucleic acid molecule is amplified and subject to PCR. In an aspect, PCR is performed on a sample previously processed using the disclosed sample preparation methodology. In yet another aspect, PCR is performed in the presence of beads, for example paramagnetic beads, after the biological sample undergoes denaturation, hybridization and capture, and washing steps.

In an aspect, the label present on a second, or third, or more, antibody is detected to thus indicate the presence of the target nucleic acid molecule. Methods for detecting various labels are known in the art. For example, a bound alkaline phosphatase conjugate can be detected by chemiluminescence with a reagent such as a LUMI-PHOS 530 reagent (Lumigen, Detroit, MI) or DR2 (Applied Biosystems, Foster City, CA) using a detector such as an E/LUMINA luminometer (Source Scientific Systems, Inc., Garden Grove, CA), an OPTOCOMP I Luminometer (MGM Instruments, Hamden, CT), or the like, such as a Veritas Microplate Luminometer by Turner Biosystems. Multiple detection techniques can also be used in sequence or in parallel. For example, the conjugate may be detected by chemiluminescence and fluorescence. In another aspect, the conjugate can be detected by chemiluminescence.

Detectors using different detection techniques for the conjugate may be reversible or irreversibly attached, for example in a modular fashion, to a machine that is capable of performing the method for determining the presence of a target nucleic acid molecule in a sample.

### Polynucleotide Probes

The polynucleotide probes are designed to hybridize or bind with the target nucleic acid molecules. In an aspect, the polynucleotide probes are designed to specifically bind to target nucleic acid molecules. In one aspect, the polynucleotide probes are about 15 bases, about 20 bases, about 25 bases, about 30 bases, about 50 bases, about 100 bases, about 250 bases, about 500 bases, about 1000 bases in length. In another aspect, the polynucleotide probes are about 15 bases or more, about 20 bases or more, about 25 bases or more, about 30 bases or more, about 50 bases or more, about 100 bases or more, about 250 bases or more, about 500 bases or more, or about 1000 bases or more in length. In another aspect, the polynucleotide probes are about 15 bases to about 25 bases, about 25 bases to about 50 bases, about 50 to about 100 bases, about 250 bases to about 500 bases, or about 1000 bases to about 5000 bases in length.

In an aspect, the polynucleotide probes are capable of hybridizing or binding to *Neisseria gonorrhoeae*, *Chlamydia trachomatis*, HPV, HPV high risk, and HPV low risk variants. In an additional aspect, the polynucleotide probes are specific for HPV and HPV high risk variants. High risk nucleic acid probes can include probes for HPV high risk types 16, 18, 31, 33, 35, 39, 45, 51, 52, 56, 58, 59, 66, 68 and 82. In other aspects the RNA or DNA probes are fragments. In an aspect, the probes are about 6 to about 8 kilobases in length, preferably about 7.5 kilobases, and may be produced using a plasmid template using a BLUESCRIPT vector. However, other plasmids, vectors and methods are known in the art and could also be used to produce the RNA probes described herein.

The probes may vary in amount from about 7.5 ng to about 60 ng per HPV type per assay, or from about 20 ng to about 45 ng per HPV type per assay, or about 30 ng of probe for each HPV type per assay is used. Thus, in one aspect the HR probes consist of or consist essentially of one or more probes for HPV high risk types 16, 18, 31, 33, 35, 39, 45, 51, 52, 56, 58, 59, 66, 68, and 82 or low risk HPV types 6, 11, 40, 43, 53, 61, 67, 69, 70, 71, 72, 81, and 83, wherein about 30 ng of each probe is used per assay for detection of the target nucleic acid molecule.

The RNA probes may be short synthetic RNA probes that specifically bind only to the target nucleic acid molecule. Examples are described in U.S. Pat. Appl. No. 12/426,076, filed on April 17, 2009.

### Cross-Reactivity

The present disclosure also provides for assay compositions, probes, and conditions wherein cross-reactivity between HPV HR probe sets and low risk HPV types is dramatically reduced when compared to the standard FDA approved HPV assay and probe set. In one aspect, the HPV HR probe set is selected from the group consisting of HPV high risk types 16, 18, 31, 33, 35, 39, 45, 51, 52, 56, 58, 59, 66, 68, and 82 or low risk HPV types 6, 11, 40, 43, 53, 61, 67, 69, 70, 71, 72, 81, and 83. Using the present assay with these HR HPV probes, cross-reactivity between low risk HPV types and high risk HPV probes is reduced. See, for example, U.S. Pat. Appl. No. 12/426,076.

The present disclosure also provides a method for determining the presence of a target nucleic acid molecule in a sample using the disclosed large volume sample preparation methods in about 30 minutes or less, about 1 hour or less, about 2 hours or less, about 2.5 hours or less, about 3 hours or less, about 3.5 hours or less, about 4 hours or less, about 5 hours or less, about 6 hours or less, about 7 hours or less, about 8 hours or less, about 12 hours or less, about 24 hours or less, in other aspects, less than about 3.5 hours for at least 10 samples using the methods discussed above.

The present disclosure also provides methods and assays for detecting cancer, for example cervical cancer, by detecting the presence of a target nucleic acid molecule, such as HPV, in a sample in about 2 hours or less, about 2.5 hours or less, about 3 hours or less, about 3.5 hours or less, about 4 hours or less, about 5 hours or less, about 6 hours or less, about 7 hours or less, about 8 hours or less, about 12 hours or less, about 24 hours or less, in other aspects, less than about 3.5 hours for at least 10 samples using the methods and assays discussed above.

It will be understood to those skilled in the art that the present invention can be carried out on a number of platforms including, but not limited to, tubes, dipsticks, microarrays, microplates, 384 well plates, other microtiter plates and microfluidic systems. It will be understood to those skilled in the art that the present, as relevant to developing countries, can utilize low technology methods such as dropper bottles, rubber bulbs, Pasteur pipettes, or squirt bottles for steps involving movement of liquid. These devices deliver relatively precise volumes within the approximate ranges that are needed for the assay. In an aspect, the methods of the disclosure do not include automatic pipettors or other battery powered or energy powered pipetting devices.

### Detection time and sensitivity

In an aspect, the biological or clinical sample is present and is capable of being isolated or detected at a concentration of about 1, about 2, about 5, about 10, about 25, about 50, about 100, about 200, about 500, about 1,000, about 5,000, about 10,000, or about 20,000, or about 100,000 target cells or copies per 1 mL of collection medium. In another aspect, the biological or clinical sample is present and is capable of being isolated or detected at a concentration of about 2 or more, about 5 or more, about 10 or more, about 25 or more, about 50 or more, about 100 or more, about 200 or more, about 500 or more, about 1,000 or more, about 5,000 or more, about 10,000 or more, or about 20,000 or more, or about 100,000 or more target cells or copies per 1 mL. In another aspect, the biological or clinical sample is present and is capable of being isolated or detected at a concentration of about 2 or less, about 5 or less, about 10 or less, about 25 or less, about 50 or less, about 100 or less, about 200 or less, about 500 or less, about 1,000 or less, about 5,000 or less, about 10,000 or less, or about 20,000 or less, or about 100,000 or less target cells or copies per 1 mL. Any biological or clinical material, for example SiHa cells, may be present in the above concentration.

In an aspect, the sample preparation methods and assays disclosed herein are capable of isolating, identifying, or detecting a concentration of about 1, about 2, about 5, about 10, about 25, about 50, about 100, about 200, about 500, about 1,000, about 5,000, about 10,000, or about 20,000, or about 100,000 target cells or copies per 50 µl or more, about 100 µl or more, about 250 µl or more, 0.5 mL or more, 1 mL or more, 2 mL or more, 5 mL or more, or 10 mL or more of collection medium in less than about 5 minutes, less than about 10 minutes, less than about 15 minutes, less than about 20 minutes, less than about 25 minutes, less than about 30 minutes, less than about 45 minutes, less than about 1 hour, less than about 2 hours, less than about 3 hours, less than about 6 hours, less than about 12 hours, or less than about 24 hours.

In another aspect, the sample preparation methods and assays disclosed herein are capable of isolating, identifying, or detecting a concentration of about 1 or more, about 2 or more, about 5 or more, about 10 or more, about 25 or more, about 50 or more, about 100 or more, about 200 or more, about 500 or more, about 1,000 or more, about 5,000 or more, about 10,000 or more, or about 20,000 or more, or about 100,000 or more target cells or copies per 50 µl or more, about 100 µl or more, about 250 µl or more, 0.5 mL or more, 1 mL or more, 2 mL or more, 5 mL or more, or 10 mL or more of collection medium in less than about 5 minutes, less than about 10 minutes, less than about 15 minutes, less than about 20 minutes, less than about 25 minutes, less than about 30 minutes, less than about 45 minutes, less than about 1 hour, less than about 2 hours, less than about 3 hours, less than about 6 hours, less than about 12 hours, or less than about 24 hours.

In another aspect, the sample preparation methods and assays disclosed herein are capable of isolating, identifying, or detecting a concentration of about 2 or less, about 5 or less, about 10 or less, about 25 or less, about 50 or less, about 100 or less, about 200 or less, about 500 or less, about 1,000 or less, about 5,000 or less, about 10,000 or less, or about 20,000 or less, or about 100,000 or less target cells or copies per 50 µl or more, about 100 µl or more, about 250 µl or more, 0.5 mL or more, 1 mL or more, 2 mL or more, 5 mL or more, or 10 mL or more of collection medium in less than about 5 minutes, less than about 10 minutes, less than about 15 minutes, less than about 20 minutes, less than about 25 minutes, less than about 30 minutes, less than about 45 minutes, less than about 1 hour, less than about 2 hours, less than about 3 hours, less than about 6 hours, less than about 12 hours, or less than about 24 hours.

In an aspect, about 10 copies or less of a target nucleic acid molecule can be isolated, identified, or detected by the methods described herein in a volume of about 1 mL to about 20 mL of collection media in a time period of about 30 minutes to about 3 hours. In an another aspect, about 10 copies or less of a target nucleic acid molecule can be detected by the methods described herein in a volume of about 1 mL or more of collection media in a time period of about 5 minutes, 10 minutes, 15 minutes, about 30 minutes, about 45 minutes, about 1 hour, about 2 hours, about 3 hours about 5 hours, about 10 hours, or about 24 hours. In other aspects, about 2 or less, about 5 or less, about 10 or less, about 25 or less, about 50 or less, about 100 or less, about 200 or less, about 500 or less, about 1,000 or less, about 5,000 or less, about 10,000 or less, or about 20,000 or less, or about 100,000 or less of a target nucleic acid molecule can be detected by the methods described herein in a volume of about 1 mL or more of collection media in a time period of about 5 to about 15 minutes, about 15 to about 30 minutes, 30 minutes to about 1 hour, about 1 hour to about 2 hours, about 2 hours to about 4 hours, and about 4 hours to about 8 hours. In an aspect, the target nucleic acid molecule is capable or binding or hybridizing to at least one HPV probe selected from the group consisting of HPV high risk types 16, 18, 31, 33, 35, 39, 45, 51, 52, 56, 58, 59, 66, 68, and 82 or low risk HPV types 6, 11, 40, 43, 53, 61, 67, 69, 70, 71, 72, 81, and 83. In another aspect, the target nucleic acid molecule is capable of binding or hybridizing to probes specific for targets of *Neisseria gonorrhoeae* or *Chlamydia trachomatis.*

In an aspect, a clinical or biological sample can be processed with the above detection sensitivities by using the disclosed sample preparation methodology in conjunction with a semi-automated or fully automated assay or instrument. For example, a clinical or biological sample may be processed using the disclosed large volume sample preparation methodology in conjunction with the assays, methods, and instruments set forth in U.S. Patent Application No. 12/508,304, U.S. Patent Application No. 12/508,306, and U.S. Patent Application No. 12/622,131.

In another aspect, the sequence specific large volume sample preparation methods described herein are capable of identifying target nucleic acid molecules with a sensitivity of 15,000 copies in a volume of 1 mL or more of collection media in less than 3 hours. Additionally, in another aspect, a sensitivity of 100 copies of HPV 16 target are detected with an input volume of 2 mL or more of collection media by hybrid capture combined with Whole Genome Amplification (WGA).

In an aspect, methods of the disclosure can include the collection and processing of patient samples in the field. In one aspect, after the samples are collected, some of the method steps are conducted at the same location where the patient samples are collected. In another aspect, all of the method steps can be conducted at the same location where the samples are collected. The location may be a village, clinic, laboratory, or communal area where individuals receive medical checkups and evaluations. The location may be permanent or temporary. In an aspect, the nucleic acid molecule is detected at a location, such as a laboratory or clinic, which is different from where the samples are taken. In an aspect, a kit is designed for use in a developing country or geographical areas where access to medical care is not readily available.

The speed of the large volume sample preparation and detection methods described herein is also beneficial in rapidly and accurately diagnosing and screening biological or clinical samples from patients in remote living areas. Often patients will travel quite a distance to visit the doctor or clinic and will not likely return for some time thereafter. Thus, it is desirable to be able to test the patient and provide results while the patient waits at the clinic. Under some circumstances, tracking down the patient to provide test results and/or treat the patient after they have left the doctor's office may be difficult.

The methods and assays of the disclosure address the need for a method of rapidly preparing large volume samples and detecting target nucleic acid molecules. The described assays provide results by identifying a target nucleic acid molecule over a short time, for example, from about 30 minutes to about 1 hour, from about 30 minutes to about 2 hours, from about 1 hour to about 2 hours, from about 1 hour to about 3 hours, or from about 2 hours to about 4 hours. In another aspect, the described methods and assays provide results in less than 15 minutes, less than 30 minutes, less than about 45 minutes, less than 1.0 hour, less than 2 hours, less than 3 hours, less than 4 hours, less than 8 hours, less than 12 hours, and less than 24 hours. Such a short turnaround time allows the doctor to provide the patient with the results and/or treatment the same day the patient is at the clinic.

### Kit/Diagnostic assay

Also provided is a large volume sample preparation kit or diagnostic assay comprising, consisting of or, or consisting essentially of:
**A.** collection medium;
**B.** denaturation reagent;
**C.** lysis buffer;
**D.** at least one polynucleotide probe;
**E.** a bead coated with an antibody; and
**F.** wash buffer.

Also provided is a large volume sample preparation kit or diagnostic assay comprising, consisting of, or consisting essentially of:
**A.** collection medium;
**B.** denaturation reagent;
**C.** lysis buffer;
**D.** wash buffer;
**E.** computer software for generating a polynucleotide probe capable of hybridizing to/capturing a target nucleic acid molecule of interest.

In an aspect, the kit or diagnostic assay may also include a resuspension buffer.

In an aspect, when the sample to be evaluated is a bodily fluid, such as blood, urine, or serum, a collection medium may be absent from the kit or diagnostic assay.

In an aspect, the kit or diagnostic assay is configured for large volume sample preparation. In an aspect, the kit or diagnostic assay is configured for sample preparation of about 50 µl or more, about 100 µl or more, about 250 µl about .5 mL or more, about .75 mL or more, about 1.0 mL or more, about 1.25 mL or more, about 1.5 mL or more, about 2.0 mL or more, about 2.5 mL or more, about 3.0 mL or more, about 5.0 mL or more, about 10 mL or more, about 15 mL or more, about 25 mL or more, about 30 mL or more, about 50 mL or more, or about 100 mL or more of any the above collection media. In an aspect, the kit or diagnostic assay, when used in a sample preparation method to detect a target nucleic acid molecule, provides detailed assay instructions regarding isolating, identifying, or detecting a concentration of about 2 or less, about 5 or less, about 10 or less, about 25 or less, about 50 or less, about 100 or less, about 200 or less, about 500 or less, about 1,000 or less, about 5,000 or less, about 10,000 or less, or about 20,000 or less, or about 100,000 or less target cells or copies per 50 µl or more, about 100 µl or more, about 250 µl or more, 0.5 mL or more, 1 mL or more, 2 mL or more, 5 mL or more, or 10 mL or more of collection medium in less than about 5 minutes, less than about 10 minutes, less than about 15 minutes, less than about 20 minutes, less than about 25 minutes, less than about 30 minutes, less than about 45 minutes, less than about 1 hour, less than about 2 hours, less than about 3 hours, less than about 6 hours, less than about 12 hours, or less than about 24 hours. In an aspect, without being limited, the detailed instructions are those found in the example protocols in Figures 9 and 10.

In an aspect, plastic tubes, for example, Eppendorf tubes, snap-cap tubes, or any other tubes capable of containing the above volumes of liquids may be included with the kit.

In another aspect, the kit or diagnostic assay, when used in a sample preparation method to detect a target nucleic acid molecule, provides detailed assay instructions regarding the conditions necessary to isolate, identify, or detect a concentration 10 copies or more of the target nucleic acid molecule are isolated in less than about 15 minutes, less than about 30 minutes, less than about 45 minutes, or less than about 1 hour. In another aspect, 50 copies or fewer of a target nucleic acid molecule are detected over a time period of about 30 minutes to about 1 hour.

Without being limited, the instructions accompanying the kit may be paper, computer software, or a link to a website for uploading the instructions.

In an aspect, the instructions indicate that no centrifugation step is used during the sample preparation. In another aspect, the instructions indicate that the sample may be amplified via PCR after the wash step with the beads present.

In an aspect, the kit or diagnostic assay can include instructions detailing, for example, the protocols set forth in Figures 9 and 10. In an aspect, the instructions included with the kit, when followed, result in the above sensitivity and completion time for copies detected/volume of solution/time.

In another aspect, the kit or diagnostic assay can be used in conjunction with the assays, methods, and instruments set forth in U.S. Patent Application No. 12/508,304, U.S. Patent Application No. 12/508,306, and U.S. Patent Application No. 12/622,131. In another aspect, the instructions accompanying the kit provide guidance on using the disclosed sample preparation methods together with an automated or semi-automated platform. In a further aspect, the kit or diagnostic assay can include tubes, pipette tips, microtiter plates, or any other mechanism for practicing the sample preparation methods described herein with the cited automated platform references.

Any of the collection medium, denaturation reagent, lysis buffer, at least one polynucleotide probe, bead, and wash buffer previously described can be used with or can accompany the kit or diagnostic assay.

The kit may also include any instructions for describing procedures associated with the disclosed methods and assays. The kit may also include a means for transcribing patient information. In an aspect, the means includes paper, a computer, or a device capable of transmitting patient information. The kit can include all the necessary components to complete the methods at the same location where the patient sample is taken.

In an aspect, the kit may include color coded reagents associated with the detection assay. The reagent vials are color coded for ease of use and can be included in a kit. The reagent bottles may also be identified by symbols, letters, or other known identifiers.

As the individual components of the kit can come together in an easy to use platform, one advantage of the kit described herein is that it provides for immediate testing of samples. This allows for rapid determination of patient results.

In an aspect, methods of the disclosure can include the collection and processing of patient samples in the field. In one aspect, after the samples are collected, some of the method steps are conducted at the same location where the patient samples are collected. In another aspect, all of the method steps can be conducted at the same location where the samples are collected. The location may be a village, clinic, laboratory, or communal area where individuals receive medical checkups and evaluations. The location may be permanent or temporary. In an aspect, the nucleic acid molecule is detected at a location, such as a laboratory or clinic, which is different from where the samples are taken. In an aspect, the kit is designed for use in a developing country or geographical areas where access to medical care is not readily available.

The following examples are illustrative only and are not intended to limit the disclosure in any way.

### EXAMPLES

### Example 1:

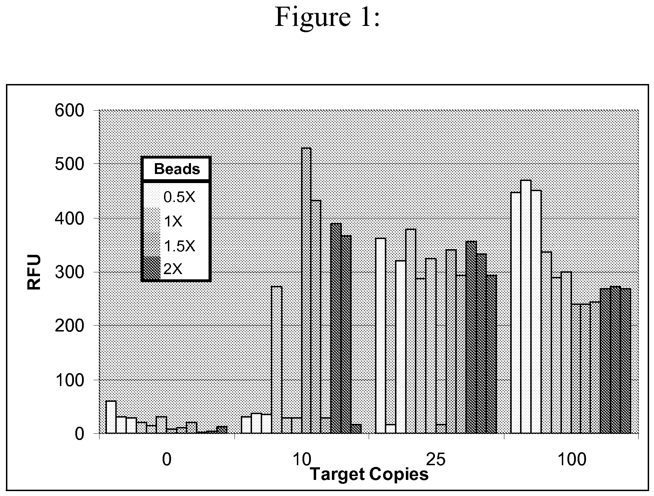

Bead concentration is tested at 0.04% in 25 µl YT blocker in 1 mL of clean PRESERVCYT collection media. The reaction takes place in 1mL of clean PRESERVCYT with 250 µl lysis buffer, 500 µl denaturation buffer, 800 µl of probe in a probe diluent, and with 2nm of synRNA. The hybridization reaction takes place for 30 minutes at room temperature. The bead concentration was tested from 0.5, 1.0, 1.5, and 2.0 times 0.04% beads in 25 µl YT. As set forth in Figure 1, background is dependent on bead concentration. Moreover, increasing bead concentration decreases background as well as raw signal, thus benefitting the signal to noise ratio (S/N).

### Example 2:

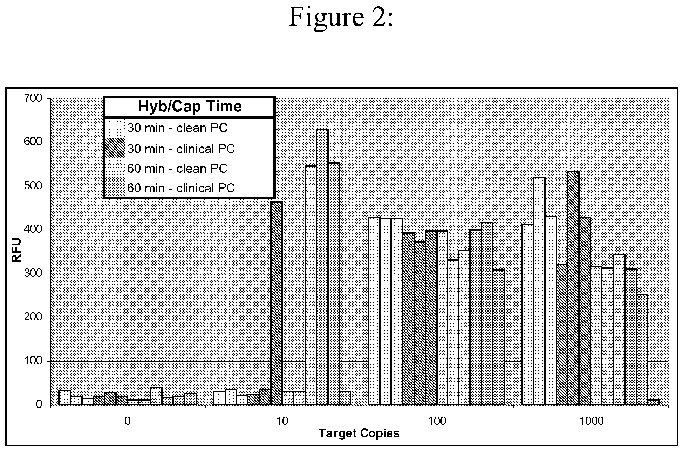

Hybrid capture large volume sample preparation with 30 minutes and 60 minutes incubation at room temperature. Bead concentration tested is 0.04% in 25 µl YT. 1 mL of clean as well as clinical PRESERVCYT collection media is tested with 0, 10, 25, and 100 copies of *Neisseria gonorrhoeae* genomic DNA. The reaction takes place in 1mL of clean or clinical PRESERVCYT media with 250 µl lysis buffer, 500 µl denaturation buffer, 800 µl of probe in probe diluent, and with 2 nm of synRNA. As set forth in Figure 2, increasing hybrid capture time to 60 minutes does not significantly benefit capture of target. For example, at 10 copies, there are fewer dropouts with 60 min hybrid capture; however no clear benefit is seen at 100 or 1000 copies. Raw signal at 100 and 1000 copies is higher with shorter incubation, with comparable background. This applies in both clean and clinical background systems.

### Example 3:

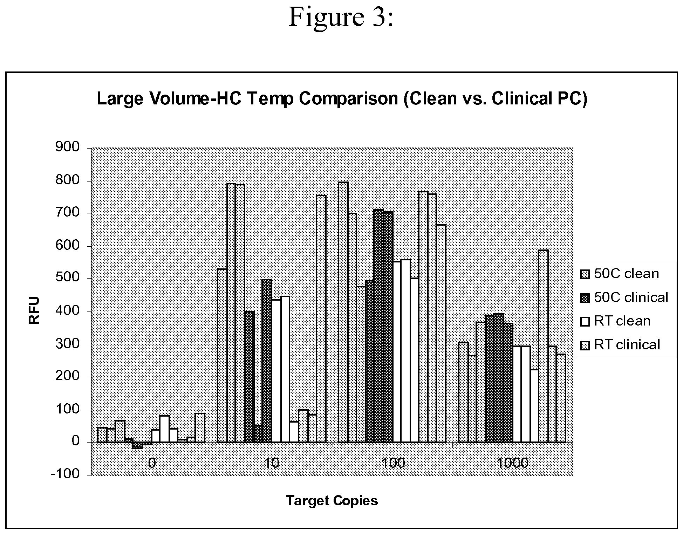

Hybrid capture sample preparation at room temperature and 50°C incubation is investigated in 1 mL of clean as well as clinical PRESERVCYT collection media with 0, 10, 25, and 100 copies of *Neisseria gonorrhoeae* genomic DNA. Bead concentration tested is 0.04% in 25 µl YT. The reaction takes place in 1 mL of clean PRESERVCYT with 250 µl lysis buffer, 500 µl denaturation buffer, 800 µl of probe in probe diluents and with 2 nm of synRNA. The hybridization reaction takes place over a 30 minute period of time. As set forth in Figure 3 and Table 1, there appears to be no significant difference in signal for clean versus clinical PRESERVCYT media above 10 copies. A large degree of variability is observed at 10 copies, however all samples in clean media at 10 copies appear to be detected. There also does not appear to be any significant difference in detection at 50°C verses room temperature.

**Table 1**

| | | | | | | | | | | |
|---|---|---|---|---|---|---|---|---|---|---|
| | **No Sample Prep Control** | | **Large Volume Sample Prep** | | | | | | | |
| | | | **H/C Temp 50C** | | | | **H/C Temp RmTmp** | | | |
| | | | **Clean PC** | | **Clinical PC** | | **Clean PC** | | **Clinical PC** | |
| | **opa** | **CTs** | **opa** | **CTs** | **opa** | **CTs** | **opa** | **CTs** | **opa** | **CTs** |
| | 22 | 89 | 13 | 106 | 16 | 102 | 12 | 78 | 5 | 77 |
| **0 Copies** | 10 | 83 | 7 | 85 | 25 | 88 | 7 | 70 | 6 | 66 |
| | 15 | 89 | 17 | 91 | 19 | 92 | 23 | 85 | 26 | 80 |
| **Avq** | 16 | 87 | 12 | 94 | 20 | 94 | 14 | 78 | 12 | 74 |
| **%CV** | 38 | 4 | 41 | 12 | 23 | 8 | 58 | 10 | 96 | 10 |
| **S/N** | **1.0** | **1.0** | **1.0** | **1.0** | **1.0** | **1.0** | **1.0** | **1.0** | **1.0** | **1.0** |
| | 30 | 119 | 101 | 101 | 268 | 95 | 364 | 79 | 33 | 92 |
| **10 Copies** | 186 | 104 | 503 | 142 | 26 | 100 | 411 | 79 | 503 | 89 |
| | 57 | 104 | 337 | 89 | 399 | 93 | 33 | 87 | 560 | 83 |
| **Avq** | 91 | 109 | 314 | 111 | 231 | 96 | 269 | 82 | 365 | 88 |
| **%CV** | 92 | 8 | 64 | 25 | 82 | 4 | 76 | 6 | 79 | 5 |
| **S/N** | **5.8** | **1.3** | **25.4** | **1.2** | **11.6** | **1.0** | **19.2** | **1.1** | **29.6** | **1.2** |
| **100 Copies** | 300 | 795 | 366 | 83 | 346 | 79 | 354 | 69 | 22 | 85 |
| | 165 | 87 | 334 | 80 | 297 | 72 | 286 | 68 | 377 | 72 |
| | 94 | 502 | 312 | 86 | 325 | 78 | 294 | 71 | 266 | 77 |
| **Avg** | 186 | 461 | 337 | 83 | 323 | 76 | 311 | 69 | 222 | 78 |
| **%CV** | 56 | 77 | 8 | 4 | 8 | 5 | 12 | 2 | 82 | 8 |
| **S/N** | **11.9** | **5.3** | **27.4** | **0.9** | **16.1** | **0.8** | **22.2** | **0.9** | **18.0** | **1.0** |
| **1000 Copies** | 397 | 110 | 371 | 95 | 392 | 87 | 263 | 62 | 255 | 59 |
| | 399 | 114 | 442 | 96 | 455 | 95 | 255 | 62 | 229 | 54 |
| | 399 | 1098 | 368 | 107 | 438 | 110 | 319 | 79 | 154 | 63 |
| **Avq** | 398 | 441 | 394 | 99 | 428 | 97 | 279 | 68 | 213 | 59 |
| **%CV** | 0 | 129 | 11 | 7 | 8 | 12 | 12 | 15 | 25 | 8 |
| **S/N** | **25.4** | **5.1** | **31.9** | **1.1** | **21.4** | **1.0** | **19.9** | **0.9** | **17.2** | **0.8** |

### Example 4:

Hybrid capture large volume sample preparation in 1 mL urine-based media as compared to 1 mL of PRESERVCYT media with the detection of 0, 10, 25, 100, 1000, and 10,000 copies of *Neisseria gonorrhoeae* genomic DNA. The bead concentration tested is 0.04% in 25 µl YT with 250 µl lysis buffer, 500 µl of denaturation buffer, 800 µl of probe in probe diluents, and with 2 nm of synRNA. The hybrid/capture reaction takes place over a 30 minute period of time.

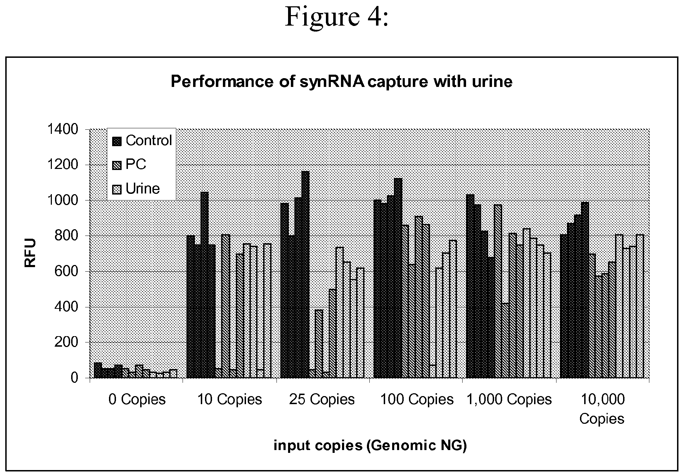

As set forth in Figure 4 and Table 2, a test of compatibility of synRNA capture in 1 mL urine (at pH 6.5) was performed. Only two dropouts are observed for the urine-based media (one at 100 copies and one at 10 copies, compared to 2 at 10 copies and 2 at 25 copies for the PRESERVCYT control). No significant hook effect is seen up to 10,000 copies. Background in urine is also quite low, resulting in relatively high S/N values.

**Table 2:**

| | **opaDv/omp/F9R6/250 Copies IC-omp-2MM All Primers 40/120nM All Probes 60nM** | | | | | | | | |
|---|---|---|---|---|---|---|---|---|---|
| | **No Prep** | | | **LV-HC Sample Prep** | | | | | |
| | | | | **Clean PC** | | | **Urine** | | |
| | **IC** | **OpaDv** | **CTs** | **IC** | **opaDv** | **CTs** | **IC** | **opaDv** | **CTs** |
| **0 Copies** | 1605 | 81 | 380 | 1017 | 49 | 135 | 776 | 35 | 99 |
| | 1284 | 50 | 138 | 859 | 35 | 102 | 700 | 23 | 93 |
| | 1164 | 50 | 120 | 793 | 70 | 152 | 414 | 33 | 134 |
| | 1248 | 74 | 481 | 1020 | 48 | 150 | 963 | 45 | 126 |
| **Avq** | 1325 | 64 | 280 | 922 | 51 | 135 | 713 | 34 | 113 |
| **%CV** | 15 | 25 | 64 | 12 | 29 | 17 | 32 | 27 | 18 |
| **S/N** | **7.0** | **1.0** | **1.0** | **4.9** | **1.0** | **1.0** | **3.8** | **1.0** | **1.0** |
| **10 Copies** | 1204 | 797 | 481 | 1085 | 50 | 158 | 769 | 755 | 457 |
| | 1093 | 751 | 336 | 960 | 807 | 166 | 865 | 742 | 116 |
| | 855 | 1048 | 171 | 930 | 48 | 127 | 912 | 45 | 111 |
| | 1170 | 751 | 133 | 566 | 697 | 77 | 773 | 752 | 95 |
| **Avg** | 1081 | 837 | 280 | 885 | 401 | 132 | 830 | 574 | 195 |
| **%CV** | 15 | 17 | 57 | 25 | 102 | 31 | 8 | 61 | 90 |
| **S/N** | **5.7** | **13.1** | **1.0** | **4.7** | **7.9** | **1.0** | **4.4** | **16.9** | **1.7** |
| **25 Copies** | 295 | 982 | 323 | 811 | 42 | 86 | 759 | 735 | 99 |
| | 1299 | 802 | 185 | 588 | 381 | 74 | 546 | 654 | 72 |
| | 1266 | 1011 | 432 | 846 | 34 | 83 | 734 | 552 | 71 |
| | 1225 | 1160 | 412 | 673 | 496 | 341 | 753 | 620 | 71 |
| **Avg** | 1021 | 989 | 338 | 730 | 238 | 146 | 698 | 640 | 78 |
| **%CV** | 48 | 15 | 33 | 16 | 99 | 89 | 15 | 12 | 18 |
| **S/N** | **5.4** | **15.5** | **1.2** | **3.9** | **4.7** | **1.1** | **3.7** | **18.8** | **0.7** |
| **100 Copies** | 317 | 997 | 244 | 567 | 861 | 105 | 683 | 70 | 89 |
| | 521 | 980 | 374 | 751 | 641 | 87 | 288 | 621 | 78 |
| | 770 | 1028 | 336 | 754 | 908 | 171 | 572 | 706 | 99 |
| | 621 | 1123 | 259 | 883 | 862 | 157 | 712 | 776 | 118 |
| **Avq** | 557 | 1032 | 303 | 739 | 818 | 130 | 564 | 543 | 96 |
| **%CV** | 34 | 6 | 20 | 18 | 15 | 31 | 34 | 59 | 18 |
| **S/N** | **3.0** | **16.2** | **1.1** | **3.9** | **16.2** | **1.0** | **3.0** | **16.0** | **0.8** |
| **1,000 Copies** | 580 | 1033 | 661 | 567 | 972 | 159 | 639 | 838 | 129 |
| | 341 | 973 | 499 | 128 | 422 | 178 | 382 | 784 | 116 |
| | 226 | 827 | 383 | 431 | 815 | 112 | 331 | 747 | 94 |
| | 301 | 678 | 398 | 551 | 751 | 111 | 437 | 703 | 90 |
| **Avg** | 362 | 878 | 485 | 419 | 740 | 140 | 447 | 768 | 107 |
| **%CV** | 42 | 18 | 26 | 49 | 31 | 24 | 30 | 7 | 17 |
| **S/N** | **1.9** | **13.8** | **1.7** | **2.2** | **14.7** | **1.0** | **2.4** | **22.6** | **0.9** |
| **10,000 Copies** | 166 | 805 | 737 | 310 | 695 | 77 | 452 | 807 | 97 |
| | 148 | 871 | 827 | 216 | 574 | 67 | 303 | 729 | 97 |
| | 263 | 919 | 902 | 263 | 584 | 87 | 436 | 744 | 184 |
| | 287 | 989 | 823 | 336 | 652 | 63 | 420 | 804 | 96 |
| **Avq** | 216 | 896 | 822 | 281 | 626 | 74 | 403 | 771 | 119 |
| **%CV** | 32 | 9 | 8 | 19 | 9 | 15 | 17 | 5 | 37 |
| **S/N** | **1.1** | **14.1** | **2.9** | **1.5** | **12.4** | **0.5** | **2.1** | **22.7** | **1.0** |

### Example 5:

A range of RNA concentration is tested in1 mL of clean PRESERVCYT collection media together with 10,000 copies of *Neisseria gonorrhoeae* genomic DNA. RNA concentrations of 0.672nM, 1.344nM, and 2.688nM are tested in Table 3. As set forth in Table 3, there does not appear to be any significant difference in either raw signal or in S/N for RNA concentrations of 0.672 nM, 1.344 nM, and 2.688 nM using the large-volume platform. The signal to noise ratio (S/N) remains at about 2.

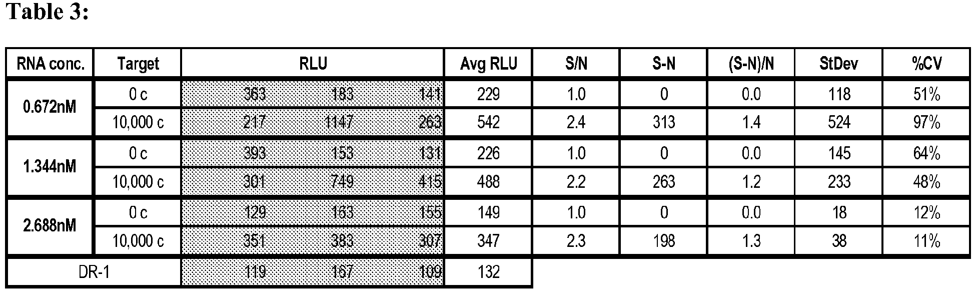

### Example 6:

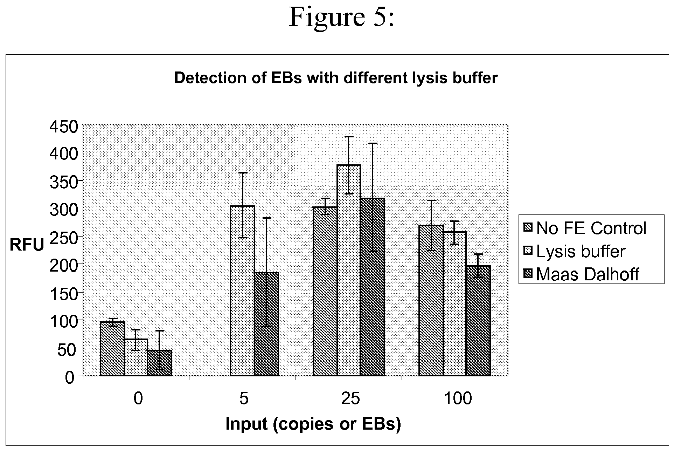

The effectiveness of a lysis buffer containing Sarkosyl, DTT, and Tween 20 is compared to the Maas-Dalhoff lysis buffer (published J.Clin.Microbiol 1994). Maas-Dalhoff lysis buffer contains Tris-HCl, SDS, Tween 20, NP-40, and Proteinase K. The lysis/denaturation step takes place at 50°C with denaturation and a lysis buffer for 30 minutes. There is no shaking present during the denaturation step. The hybrid capture step takes place at about 50°C for about 30 minutes with shaking at 900 rpm. The hybrid capture step is monoplex capture using 500 base pair synRNA probes at a concentration of 2.0 nM with 0.00039% beads. The experiment is performed with the monoplex tHDA model, using either CT genomic with Omp7 primers and omp_TYE probe, or NG genomic with OpaDv primers and OpaDb1_Tye probe. As set forth in Figure 5, usingCT EBs for target, the lysis buffer containing Sarkosyl, DTT, and Tween 20 exhibits a higher S/N ratio than experiments performed with Maas-Dalhoff lysis buffer.

### Example 7:

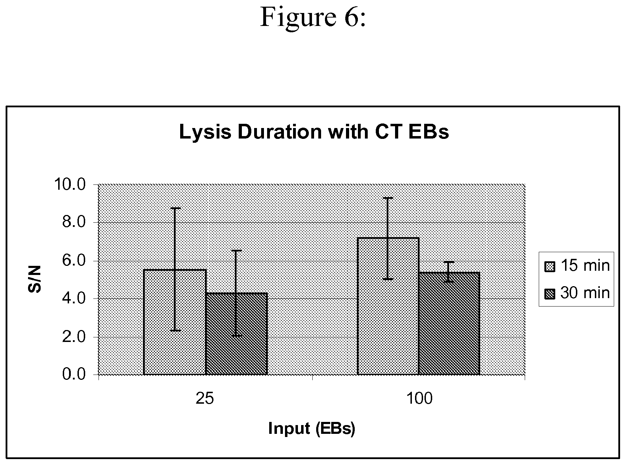

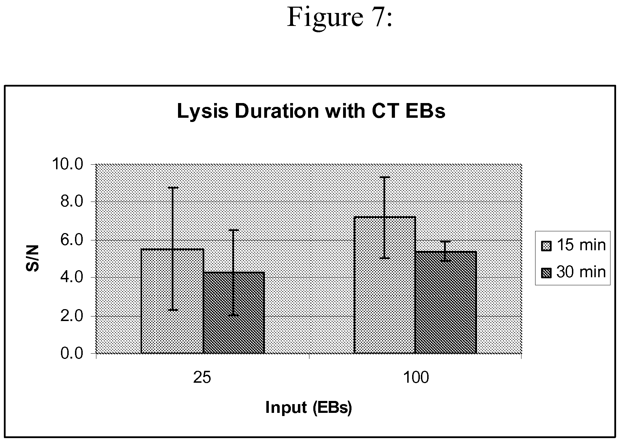

A lysis buffer containing Sarkosyl, DTT, and Tween 20 is evaluated on a large volume platform over the course of 15 minute and 30 minutes incubation times at 50°C. There is no shaking present during the denaturation/lysis step. The hybrid capture step takes place at 50°C for 30 minutes with shaking at 900 rpm. The hybrid capture step is monoplex capture using 500 base pair synRNA probes at a concentration of 2.0 nM with 0.00039% beads. The experiment is performed with the monoplex tHDA model, using either *Chlamydia trachomatis* genomic with Omp7 primers and omp_TYE probe, or *Neisseria gonorrhoeae* genomic with OpaDv primers and OpaDb1_Tye probe. As set forth in Figure 6, the Lysis/dantuaration step was evaluated with *Chlamydia trachomatis* EBs as a target at 15 minutes and 30 minutes. As set forth in Figure 7, the lysis/denaturation step was evaluated with NG cells as a target at 15 minutes and 30 minutes. the lysis buffer containing Sarkosyl, DTT, and Tween 20 exhibits a higher S/N ratio than experiments performed with the Maas-Dalhoff lysis buffer. Decreasing denaturation/lysis time does not have a negative impact on S/N. One dropout was seen with target input of 25 EB for both 15 and 30 minute lysis.

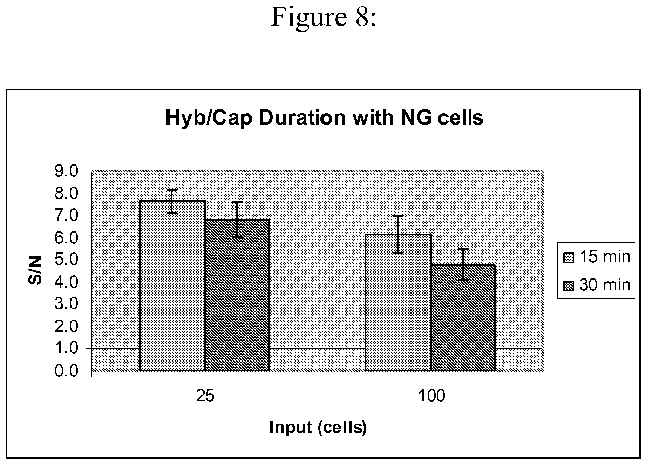

Under the above conditions, the hybrid/capture step is evaluated at 15 minutes and 30 minutes. As set forth in Figure 8, decreasing hybridization/capture time does not have a negative impact on signal to noise ratio. The overall decrease in S/N is caused by a slight increase in background at 30 minutes hybrid/capture. Raw signal for both 25 and 100 cells input is comparable.

## Claims

1. A method of isolating a target nucleic acid molecule from a large volume sample, the method comprising:
(a) suspending a biological sample in 1 mL or more of a collection media;
(b) denaturing and lysing the biological sample by adding a denaturation agent and lysis buffer to the suspended biological sample;
(c) hybridizing a target nucleic acid molecule to at least one polynucleotide probe;
(d) capturing the hybridized target nucleic acid molecule on a support;
(e) washing the captured hybrid-support with wash buffer to separate the hybridized target nucleic acid molecule captured on the support from the sample, thereby isolating the target nucleic acid molecule; and
(f) resuspending the washed captured hybrid-support in a resuspension buffer;
wherein said method does not include a centrifugation step, the large volume sample is 0.5 mL or more, the denaturing and lysing step (b) is complete in less than 10 minutes and the combination of the hybridizing step (c) and the capturing step (d) is complete in less than 25 minutes and 10 copies or more of the target nucleic acid molecule are isolated if present in the sample in less than 1 hour.

2. The method of claim 1, having one of the following characteristics:
a) 10 copies or more of the target nucleic acid molecule are isolated in less than 30 minutes;
b) 10 copies or more of the target nucleic acid molecule are isolated in less than 15 minutes;
c) wherein said denaturing and lysing step (b) is complete in less than 7.5 minutes and the combination of the hybridizing step (c) and the capturing step (d) is complete in less than 22.5 minutes; or
d) said denaturing and lysing step (b) is complete in less than 5 minutes and the combination of the hybridizing step (c) and the capturing step (d) is complete in less than 15 minutes.

3. The method of claim 1, having one of the following characteristics:
a) said collection media comprises 0.5% to 2.0% NP-40, 0.10% to 0.40% sodium deoxycholate, 25 mM to 75 mM Tris- HCl, 10 mM to 50 mM EDTA, 50 mM to 200 mM NaCl, and 0.01% to 0.10% sodium azide; or
b) said collection media is selected from the group consisting of PRESERVCYT, STM, and SUREPATH.

4. The method of claim 1, wherein step (e) comprises allowing the captured hybrid-support complex to form a pellet and washing the captured hybrid-support with wash buffer.

5. The method of claim 1, **wherein** method steps (a) - (e) are completed in 20 minutes to 40 minutes.

6. The method of claim 1, having one of the following characteristics:
a) the target nucleic acid molecule is present in the collection medium at a concentration of less than 1pg/ml, less than 0.75pg/ml, less than 0.5pg/ml, less than 0.25pg/ml or less than 0.2pg/ml, or
b) the target nucleic acid is present in the collection medium at a concentration of 10,000 copies/ml or fewer, 1,000 copies/ml or fewer, 100 copies/ml or fewer or 10 copies/ml or fewer.

7. The method of claim 1, **wherein** the biological sample is a cervical cell.

8. The method of claim 1, **wherein** the target nucleic acid molecule is from *C. trachomatis* or the target nucleic acid molecule is from *N. gonorrhoeae.*

9. A method for isolating and detecting the presence of a target nucleic acid molecule from a large sample volume, the method comprising:
(a) isolating the target nucleic acid molecule with a method according to claim 1; and
(b) performing a PCR on the isolated target nucleic acid molecule.

## Patentansprüche

1. Verfahren zum Isolieren eines Zielnukleinsäuremoleküls aus einer großvolumigen Probe, wobei das Verfahren folgendes umfasst:
(a) Suspendieren einer biologischen Probe in 1 ml oder mehr eines Sammelmediums;
(b) Denaturieren und Lysieren der biologischen Probe durch Zugabe eines Denaturierungsagens und Lysepuffer zu der suspendierten biologischen Probe;
(c) Hybridisieren eines Zielnukleinsäuremoleküls an wenigstens eine Polynukleotidsonde;
(d) Fangen des hybridisierten Zielnukleinsäuremoleküls an einem Träger;
(e) Waschen des gefangenen Hybrid-Trägers mit Waschpuffer, um das an dem Träger gefangene hybridisierte Zielnukleinsäuremolekül von der Probe zu trennen, wodurch das Zielnukleinsäuremolekül isoliert wird; und
(f) Resuspendieren des gewaschenen gefangenen Hybrid-Trägers in einem Resuspensionspuffer;
wobei das Verfahren keinen Zentrifugationsschritt aufweist, die großvolumige Probe 0,5 ml oder mehr hat, der Schritt des Denaturierens und Lysierens (b) in weniger als zehn Minuten abgeschlossen ist, und die Kombination aus dem Hybridisierungsschritt (c) und dem Fangschritt (d) in weniger als 25 Minuten abgeschlossen ist, und zehn oder mehr Kopien des Zielnukleinsäuremoleküls, sofern in der Probe vorhanden, innerhalb von weniger als einer Stunde isoliert werden.

2. Verfahren nach Anspruch 1, mit einer der folgenden Eigenschaften:
a) zehn oder mehr Kopien des Zielnukleinsäuremoleküls werden innerhalb von weniger als 30 Minuten isoliert;
b) zehn oder mehr Kopien des Zielnukleinsäuremoleküls werden innerhalb von weniger als 15 Minuten isoliert;
c) wobei der Schritt des Denaturierens und Lysierens (b) in weniger als siebeneinhalb Minuten abgeschlossen ist, und die Kombination aus dem Hybridisierungsschritt (c) und dem Fangschritt (d) in weniger als 22,5 Minuten abgeschlossen ist; oder
d) der Schritt des Denaturierens und Lysierens (b) ist in weniger als fünf Minuten abgeschlossen, und die Kombination aus dem Hybridisierungsschritt (c) und dem Fangschritt (d) ist in weniger als 15 Minuten abgeschlossen.

3. Verfahren nach Anspruch 1, mit einer der folgenden Eigenschaften:
a) das Sammelmedium umfasst 0,5% bis 2,0% NP-40, 0,10% bis 0,40% Natriumdeoxycholat, 25 mM bis 75 mM Tris-HCl, 10 mM bis 50 mM EDTA, 50 mM bis 200 mM NaCl und 0,01% bis 0,10% Natriumazid; oder
b) das Sammelmedium ist ausgewählt aus der Gruppe bestehend aus PRESERVCYT, STM und SUREPATH.

4. Verfahren nach Anspruch 1, wobei Schritt (e) umfasst, es dem gefangenes Hybrid-Träger-Komplex zu ermöglichen, ein Pellet zu bilden, und Waschen des gefangenes Hybrid-Trägers mit Waschpuffer.

5. Verfahren nach Anspruch 1, wobei die Schritte (a) - (e) in 20 Minuten bis 40 Minuten abgeschlossen sind.

6. Verfahren nach Anspruch 1, mit einer der folgenden Eigenschaften:
a) das Zielnukleinsäuremolekül ist in dem Sammelmedium in einer Konzentration von weniger als 1 pg/ml, weniger als 0,75 pg/ml, weniger als 0,5 pg/ml, weniger als 0,25 pg/ml oder weniger als 0,2 pg/ml vorhanden; oder
b) das Zielnukleinsäuremolekül ist in dem Sammelmedium in einer Konzentration von 10.000 Kopien/ml oder niedriger, 1.000 Kopien/ml oder weniger, 100 Kopien/ml oder weniger oder 10 Kopien/ml oder weniger vorhanden.

7. Verfahren nach Anspruch 1, wobei die biologische Probe eine Zervixzelle ist.

8. Verfahren nach Anspruch 1, wobei das Zielnukleinsäuremolekül von *C. trachomatis* stammt, oder wobei das Zielnukleinsäuremolekül von *N. gonorrhoeae* stammt.

9. Verfahren zum Isolieren und Detektieren des Vorhandenseins eines Zielnukleinsäuremoleküls aus einer großvolumigen Probe, wobei das Verfahren folgendes umfasst:
(a) Isolieren des Zielnukleinsäuremoleküls mit einem Verfahren nach Anspruch 1; und
(b) Durchführen einer PCR an dem isolierten Zielnukleinsäuremolekül.

## Revendications

1. Procédé d'isolement d'une molécule d'acide nucléique cible à partir d'un échantillon de grand volume, le procédé comprenant les étapes consistant à :
(a) mettre en suspension un échantillon biologique dans 1 ml ou plus d'un milieu de recueil ;
(b) dénaturer et lyser l'échantillon biologique en ajoutant un agent de dénaturation et un tampon de lyse à l'échantillon biologique mis en suspension ;
(c) hybrider une molécule d'acide nucléique cible sur au moins une sonde de polynucléotide ;
(d) capturer la molécule d'acide nucléique cible hybridée sur un support ;
(e) laver le complexe support-hybride capturé avec un tampon de lavage pour séparer la molécule d'acide nucléique cible hybridée capturée sur le support de l'échantillon, en isolant ainsi la molécule d'acide nucléique cible ; et
(f) remettre en suspension le complexe support-hybride capturé lavé dans un tampon de remise en suspension ;
où ledit procédé n'inclut pas d'étape de centrifugation, l'échantillon de grand volume a un volume de 0,5 ml ou plus, l'étape de dénaturation et de lyse (b) est réalisée en moins de 10 minutes et la combinaison de l'étape d'hybridation (c) et de l'étape de capture (d) est réalisée en moins de 25 minutes et 10 copies ou plus de la molécule d'acide nucléique cible sont isolées si elles sont présentes dans l'échantillon en moins de 1 heure.

2. Procédé selon la revendication 1, présentant l'une des caractéristiques suivantes :
a) 10 copies ou plus de la molécule d'acide nucléique cible sont isolées en moins de 30 minutes ;
b) 10 copies ou plus de la molécule d'acide nucléique cible sont isolées en moins de 15 minutes ;
c) ladite étape de dénaturation et de lyse (b) est réalisée en moins de 7,5 minutes et la combinaison de l'étape d'hybridation (c) et de l'étape de capture (d) est réalisée en moins de 22,5 minutes ; ou
d) ladite étape de dénaturation et de lyse (b) est réalisée en moins de 5 minutes et la combinaison de l'étape d'hybridation (c) et de l'étape de capture (d) est réalisée en moins de 15 minutes.

3. Procédé selon la revendication 1, présentant l'une des caractéristiques suivantes :
a) ledit milieu de recueil comprend de 0,5% à 2,0 % de NP-40, de 0,10 % à 0,40 % de désoxycholate de sodium, de 25 mM à 75 mM de Tris-HCl, de 10 mM à 50 mM d'EDTA, de 50 mM à 200 mM de NaCl, et de 0,01 % à 0,10 % d'azoture de sodium ; ou
b) ledit milieu de recueil est sélectionné parmi le groupe constitué de PRESERVCYT, de STM et de SUREPATH.

4. Procédé selon la revendication 1, dans lequel l'étape (e) comprend le fait de laisser le complexe support-hybride capturé former une pastille et de laver le complexe support-hybride capturé avec un tampon de lavage.

5. Procédé selon la revendication 1, **dans lequel** les étapes de procédé (a) à (e) sont réalisées en un laps de temps compris entre 20 minutes et 40 minutes.

6. Procédé selon la revendication 1, présentant l'une des caractéristiques suivantes :
a) la molécule d'acide nucléique cible est présente dans le milieu de recueil à une concentration inférieure à 1 pg/ml, inférieure à 0,75 pg/ml, inférieure à 0,5 pg/ml, inférieure à 0,25 pg/ml ou inférieure à 0,2 pg/ml, ou
b) l'acide nucléique cible est présent dans le milieu de recueil à une concentration de 10 000 copies/ml ou moins, de 1 000 copies/ml ou moins, de 100 copies/ml ou moins, ou de 10 copies/ml ou moins.

7. Procédé selon la revendication 1, **dans lequel** l'échantillon biologique est une cellule cervicale.

8. Procédé selon la revendication 1, **dans lequel** la molécule d'acide nucléique cible provient de *C. trachomatis* ou la molécule d'acide nucléique cible provient de *N. gonorrhoeae.*

9. Procédé d'isolement et de détection de la présence d'une molécule d'acide nucléique cible à partir d'un échantillon de grand volume, le procédé comprenant les étapes consistant à :
a) isoler la molécule d'acide nucléique cible à l'aide d'un procédé selon la revendication 1 ; et
b) réaliser une PCR sur la molécule d'acide nucléique cible isolée.
